# EUROPEAN PATENT SPECIFICATION

(11) **EP 4 042 166 B1**
(45) Date of publication and mention of the grant of the patent: **09.04.2025**
(21) Application number: 20793210.4
(22) Date of filing: 28.09.2020
(51) Int. Cl.: G01N 33/68, C07K 16/24

(54) **COMPOSITIONS AND METHODS FOR AN IL-17 TARGET ENGAGEMENT ASSAY WITH SMALL MOLECULE MODULATORS**
ZUSAMMENSETZUNGEN UND VERFAHREN FÜR IL-17-ZIELBEKÄMPFUNGS-ASSAY MIT KLEINMOLEKULAREN MODULATOREN
COMPOSITIONS ET MÉTHODES DE DOSAGE D'IMPLICATION CIBLE D'IL-17 AVEC DES MODULATEURS À PETITES MOLÉCULES

(30) Priority: 30.09.2019 US 201962908195 P; 12.01.2020 US 202062960031 P; 14.09.2020 US 202063077978 P
(43) Date of publication of application: 17.08.2022
(73) Proprietor: Janssen Pharmaceutica NV, 2340 Beerse (BE)
(72) Inventor: BLEVITT, Jonathan, M., San Diego, CA 92121 (US); DEPRIMO, Samuel, E., San Diego, CA 92121 (US); STRASNER, Amy, San Diego, CA 92121 (US); LEUNG, Wai-Ping, San Diego, CA 92121 (US); DE LEON-TABALDO, Aimee, Rose, San Diego, CA 92121 (US); XUE, Xiaohua, San Diego, CA 92121 (US); GOLDBERG, Steven, San Diego, CA 92121 (US)
(74) Representative: Carpmaels & Ransford LLP
(86) International application number: PCT/US2020/053102
(87) International publication number: WO 2021/067191

(56) References cited:
- DEFORGE L E ET AL: "Evaluation of heterophilic antibody blocking agents in reducing false positive interference in immunoassays for IL-17AA, IL-17FF, and IL-17AF", JOURNAL OF IMMUNOLOGICAL METHODS, ELSEVIER SCIENCE PUBLISHERS B.V.,AMSTERDAM, NL, vol. 362, no. 1-2, 31 October 2010 (2010-10-31), pages 70 - 81, XP027509592, ISSN: 0022-1759, [retrieved on 20100909], DOI: 10.1016/J.JIM.2010.09.004
- PENG KUN ET AL: "Measurement of IL-17AA and IL-17FF as Pharmacodynamic Biomarkers to Demonstrate Target Engagement in the Phase I Study of MCAF5352A", THE AAPS JOURNAL, SPRINGER INTERNATIONAL PUBLISHING, CHAM, vol. 21, no. 1, 13 December 2018 (2018-12-13), pages 1 - 9, XP036665454, DOI: 10.1208/S12248-018-0280-Z

## Description

### BACKGROUND

Interleukin-17A (IL-17 or IL-17A) is a cytokine secreted by activated Th17 cells, CD8+ T cells, γδ T cells, and NK cells in response to cytokines such as interleukin-23 (IL-23) and Transforming Growth Factor beta (TGF-β). IL-17 regulates production of mediators such as antimicrobial peptides, pro-inflammatory cytokines, and chemokines from multiple cell types including epithelia cells, fibroblasts, and synoviocytes that are involved in the recruitment of neutrophils and pathology of tissue damage in inflammation or in host defense. IL-17 also synergizes with other cytokines, such as TNF-α and IL-Iβ to potentiate the pro-inflammatory environment.

Due to its involvement in immune regulatory functions, inhibitors of IL-17 (or modulators of IL-17) are being investigated as possible treatments for various autoimmune diseases. Anti-IL-17 mAbs are validated clinically for the treatment of psoriasis, psoriatic arthritis, ankylosing spondylitis and have demonstrated proof of concept for the treatment of multiple sclerosis. While no oral small molecule modulators of IL-17 have progressed into clinical trials yet, they remain an attractive area for discovery as their development may broaden treatment options for many patients without access to biologics. (*e.g.,* S. Liu et al. Scientific Reports, 6, 30859, https://doi.org/10.1038/srep30859).

Despite the clinical utility of large molecule modulators of IL-17, and the emergence of small molecule modulators of IL-17 in the pharmaceutical industry, there are currently no target engagement assays that have been reported, which can measure the binding activity between IL-17 and small molecule modulators thereof. That is, currently available immunoassay kits can only measure the level of total IL-17 (including both modulator-bound IL-17 and free IL-17) in samples with IL-17 and small modulators of IL-17 present. There are, however, no assays for specifically detecting only free IL-17 in the presence of a small molecule modulator of IL-17 to determine target engagement. Accordingly, information on levels of IL-17 target engagement by a small molecule modulator is lacking.

Moreover, although disease related biomarkers have been used to monitor anti-IL-17 therapeutic effects in patients, no biomarkers have been developed to monitor anti-IL-17 effects in the healthy subjects who are often included at early stages of clinical evaluation. A measurement that could demonstrate the target engagement of an IL-17 small molecule modulator with IL-17 in a subject (e.g., a human) is important for progressing SM modulators through clinical studies.

DeForge et al, J Immunol Methods. 2010 Oct 31;362(1-2):70-81 describes evaluation of heterophilic antibody blocking agents in reducing false positive interference in immunoassays for IL-17AA, IL-17FF, and IL-17AF. Peng et al, AAPS J. 2018 Dec 13;21(1):9 describes measurement of IL-17AA and IL-17FF as pharmacodynamic biomarkers to demonstrate Target Engagement in the Phase I Study of MCAF5352A.

Thus, there is a need for method(s) to assess IL-17 engagement by SM modulators in a sample and to study clinically relevant parameters such as pharmacokinetic (PK) and pharmacodynamic (PD) information, which in turn, may aid in the determination of an optimal dosage for a safe and efficacious therapy.

### BRIEF SUMMARY

In a general aspect, the application relates to a method of determining an amount of free IL-17 (*i.e.,* IL-17 that is not bound to a SM modulator(s)) in a sample comprising IL-17 and the SM modulator.

Provided herein is a method of determining an amount of free IL-17 in a sample comprising IL-17 and a small molecule (SM) modulator of IL-17, the method comprising:
i. contacting the sample with a first capture antibody to form a mixture comprising a first complex comprising the first capture antibody and the IL-17 unbound to the SM modulator, and a second complex comprising the first capture antibody and the IL-17 bound to the SM modulator;
ii. contacting the mixture from step i) with a first detection agent to thereby form a third complex comprising the first capture antibody, the IL-17 unbound to the SM modulator, and the first detection agent, but not a fourth complex comprising the first capture antibody, the IL-17 bound to the SM modulator, and the first detection agent; and
iii. determining the amount of the free IL-17 by measuring the amount of the first detection agent in the third complex,
wherein the SM modulator is selected from the group consisting of Compounds #1-6 having the following structures and pharmaceutically acceptable salts thereof: 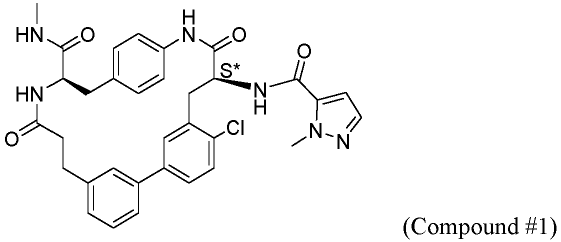 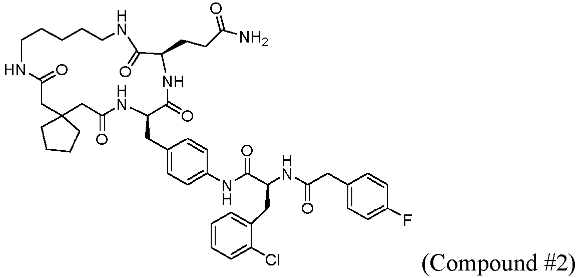 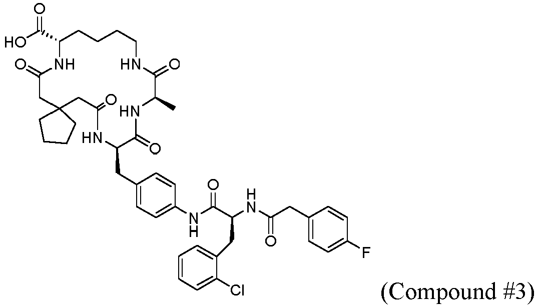 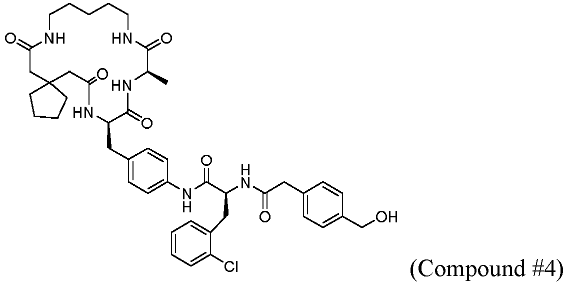 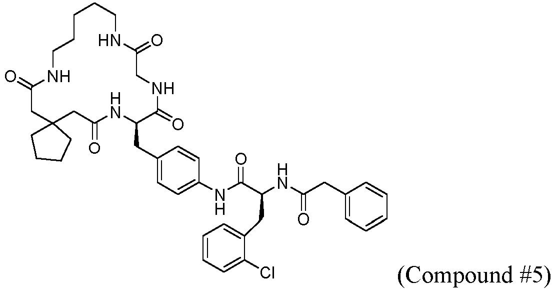 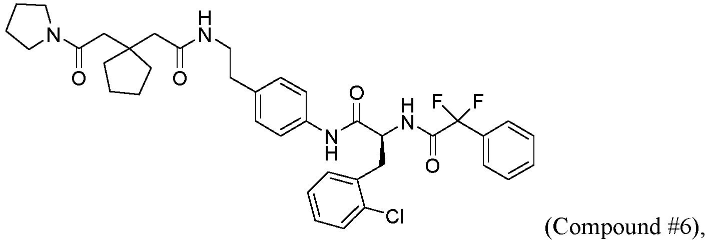 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

In one embodiment of the method, no wash step is performed before contacting the mixture from step i) with the first detection agent.

Further provided herein is method of determining the target engagement of a SM modulator of IL-17 in a subject, comprising a) determining the amount of free IL-17 in a sample from the subject before administration of the SM modulator using the method of claim 1; b) determining the amount of free IL-17 in a sample from the subject after administration of the SM modulator using the method of claim 1; and c) comparing the amounts of free IL-17 determined from steps a) and b).

Yet further provided herein is a method of determining the target engagement of a SM modulator of IL-17 in a subject, comprising a) determining the amount of free IL-17 in a sample from the subject at two or more time points after the administration of the SM modulator using the method of claim 1; and b) comparing the amounts of free IL-17 at the two or more time points determined from step a).

Yet further provided herein is a method of determining an amount of modulator-bound IL-17 in a sample comprising IL-17 and a SM modulator of IL-17, the method comprising:
i. contacting a first portion of the sample with a first capture antibody to form a first mixture comprising a first complex comprising the first capture antibody and the IL-17 unbound to the SM modulator, and a second complex comprising the first capture antibody and the IL-17 bound to the SM modulator;
ii. contacting the first mixture from step i) with a first detection agent to thereby form a third complex comprising the first capture antibody, the IL-17 unbound to the SM modulator, and the first detection agent, but not a fourth complex comprising the first capture antibody, the IL-17 bound to the SM modulator, and the first detection agent;
iii. contacting a second portion of the sample with a second capture antibody to form a second mixture comprising a fifth complex comprising the second capture antibody and the IL-17 unbound to the SM modulator, and a sixth complex comprising the second capture antibody and the IL-17 bound to the SM modulator;
iv. contacting the second mixture with a second detection agent to thereby form a seventh complex comprising the second capture antibody, the IL-17 unbound to the SM modulator, and the second detection agent and an eighth complex comprising the second capture antibody, the IL-17 bound to the SM modulator, and the second detection agent;
v. determining the amount of the free IL-17 by measuring the amount of the first detection agent in the third complex;
vi. determining a total amount of the IL-17 by measuring the total amount of the second detection agent in the seventh complex and the eighth complex; and
vii. determining the amount of the modulator-bound IL-17 in the sample by subtracting the amount of the free IL-17 determined in step (v) from the total amount of the IL-17 in step (vi),
wherein the SM modulator is selected from the group consisting of Compounds #1-6 having the following structures and pharmaceutically acceptable salts thereof: 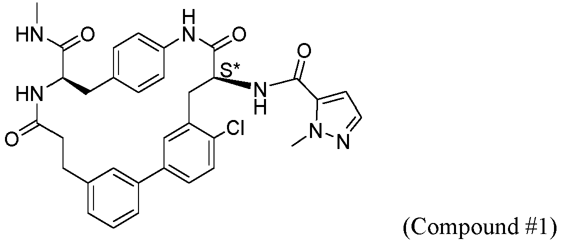 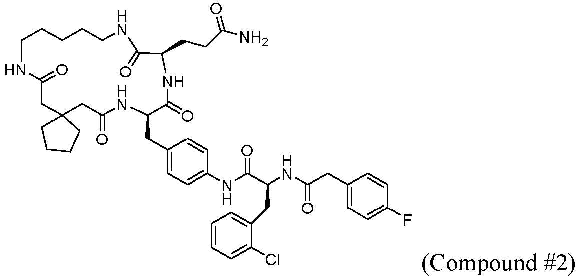 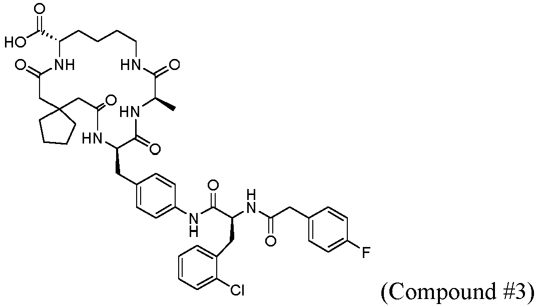 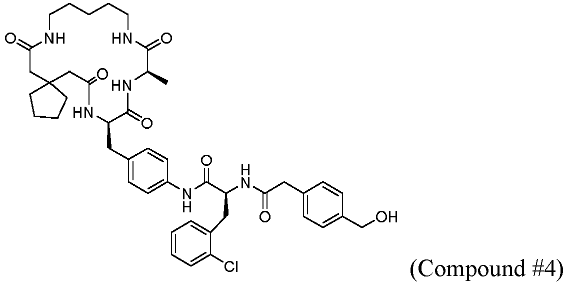 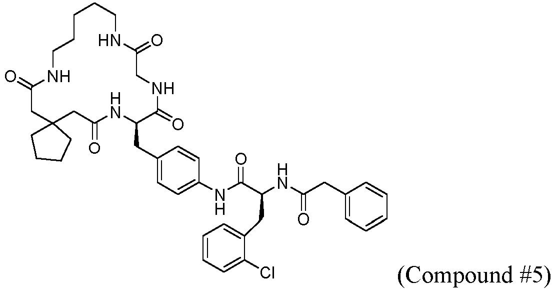 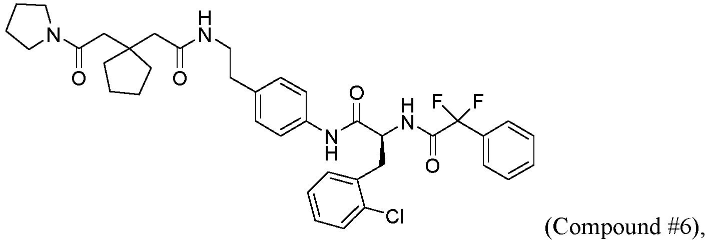 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

Yet further provided herein is a method of determining the target engagement of a SM modulator of IL-17 in a subject, comprising determining the amount of modulator-bound IL-17 in a sample from the subject after administration of the SM modulator using the method described above.

In a further embodiment of any of the methods described above, the SM modulator is selected from the group consisting of compounds #2-6 having the following structures and pharmaceutically acceptable salts thereof:

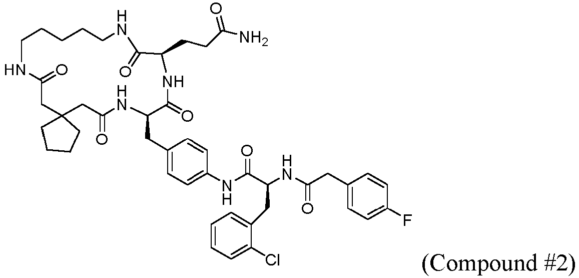

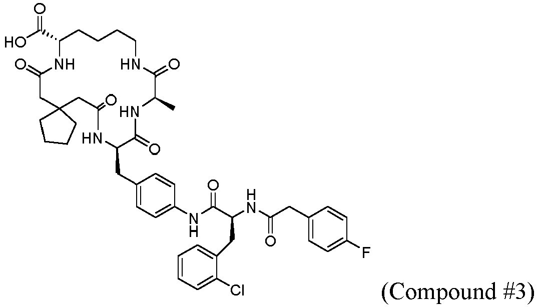

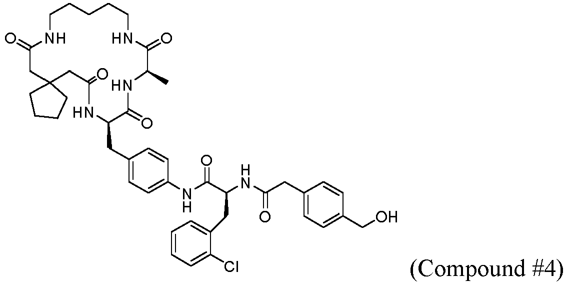

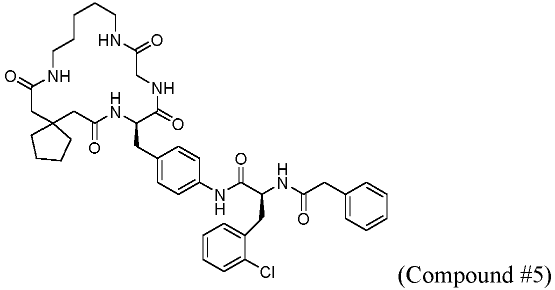

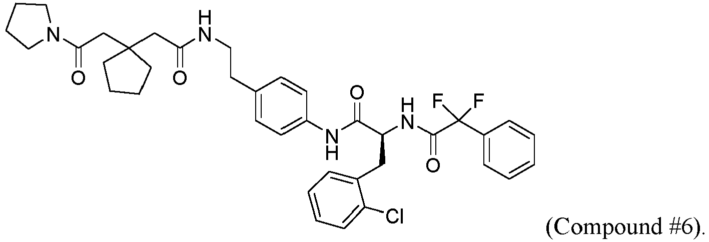

In a yet further embodiment of any of the methods described above, the first and/or the second detection agent is labeled with a detectable label, more preferably, the label is an enzyme or biotin.

In a yet further embodiment of any of the methods described above, the second capture antibody and the second detection agent are selected from the pairs in the following table:

| **Second capture antibody** | | **Second detection agent** | |
|---|---|---|---|
| **Company** | **CAT#** | **Company** | **CAT#** |
| R&D Systems, Inc. | Capture antibody of DuoSet ELISA kit DY317 | R&D Systems, Inc. | Detection antibody of DuoSet ELISA kit DY317 |
| R&D Systems, Inc. | Capture antibody of DuoSet ELISA kit DY317 | R&D Systems, Inc. | Detection antibody of human IL-17 HS ELISA Kit HS170 |
| R&D Systems, Inc. | Capture antibody of DuoSet ELISA kit DY317 | CovalAb R&D (Antibodies Online) | antibody ABIN1724556 |
| R&D Systems, Inc. | Capture antibody of DuoSet ELISA kit DY317 | Creative Diagnostics | Anti-IL 17A monoclonal antibody CABT-ZC1184 |
| R&D Systems, Inc. | Capture antibody of DuoSet ELISA kit DY317 | Mabtech AB | Anti-human IL-17 antibody 3520-3-250 |
| R&D Systems, Inc. | Capture antibody of DuoSet ELISA kit DY317 | MyBioSource (MBS) | Anti-IL-17A antibody MBS592444 |
| R&D Systems, Inc. | Capture antibody of DuoSet ELISA kit DY317 | MyBioSource (MBS) | Anti-IL-17A antibody MBS592430 |
| R&D Systems, Inc. | Capture antibody of DuoSet ELISA kit DY317 | MyBioSource (MBS) | Anti-IL-17A antibody MBS689609 |
| R&D Systems, Inc. | Capture antibody of DuoSet ELISA kit DY317 | MyBioSource (MBS) | Anti-IL-17A antibody MBS592432 |
| R&D Systems, Inc. | Capture antibody of DuoSet ELISA kit DY317 | NOVUS Biologicals LLC | IL-17/IL-17A antibody DDX0336B-100 |
| R&D Systems, Inc. | Capture antibody of DuoSet ELISA kit DY317 | Antibodies Online | ABIN786722 |
| R&D Systems, Inc. | Capture antibody of DuoSet ELISA kit DY317 | OriGene Technologies Inc. | DDX0334B-50 |
| R&D Systems, Inc. | Capture antibody of DuoSet ELISA kit DY317 | OriGene Technologies Inc. | DDX0335B-50 |
| R&D Systems, Inc. | Capture antibody of DuoSet ELISA kit DY317 | Rockland Immunochemicals Inc. | IL-17A biotin conjugated antibody 209-306-B32 |

In a yet further embodiment of the any of the methods described above, the second capture antibody and the second detection agent are capture antibody and detection antibody of DuoSet ELISA kit DY317 (from R&D Systems, Inc.), respectively.

In a yet further embodiment of any of the methods described above, the sample is a biological sample obtained from a subject under an *ex vivo* or *in vivo* treatment with the SM modulator. The subject may be a mammal, preferably human. The biological sample may be selected from the group consisting of a sample prepared from cells, tissues, or a serum, plasma or another biological fluid sample.

In a yet further embodiment of any of the methods described above, the method has a detection sensitivity with Lower Limit of Quantification (LLOQ) at about 0.01, or about 0.1, or about 0.25, or about 0.5, or about 0.75, or about 1 pg/ml of the IL-17 in the sample.

Yet further provided herein is a kit for determining an amount of free IL-17 in a sample comprising IL-17 and a SM modulator of IL-17, the kit comprising:
i. a first capture antibody, wherein upon contact with the sample, the first capture antibody forms a first complex comprising the first capture antibody and the IL-17 unbound to the SM modulator, and a second complex comprising the first capture antibody and the IL-17 bound to the SM modulator; and
ii. a first detection agent, wherein upon contact with the first complex and the second complex, the first detection agent, forms a third complex comprising the first capture antibody, the IL-17 unbound to the SM modulator, and the first detection agent, but not a fourth complex comprising the first capture antibody, the IL-17 bound to the SM modulator, and the first detection agent,
wherein the SM modulator is selected from the group consisting of Compounds #1-6 having the following structures and pharmaceutically acceptable salts thereof: 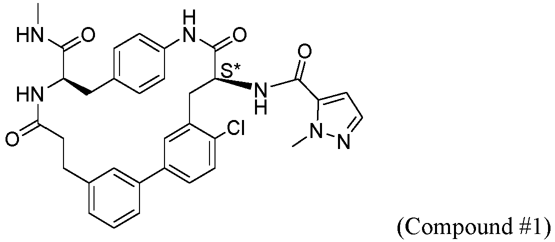 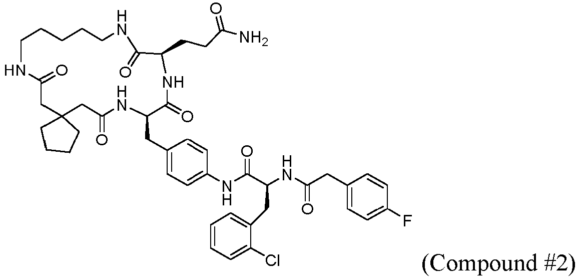 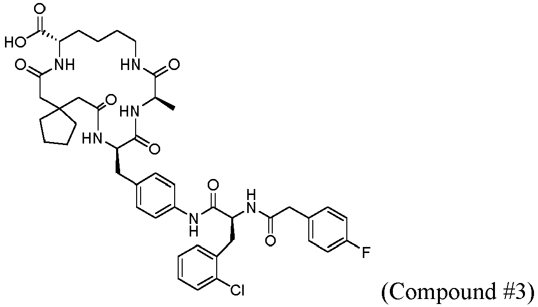 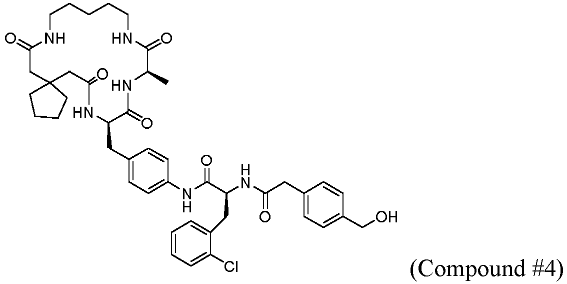 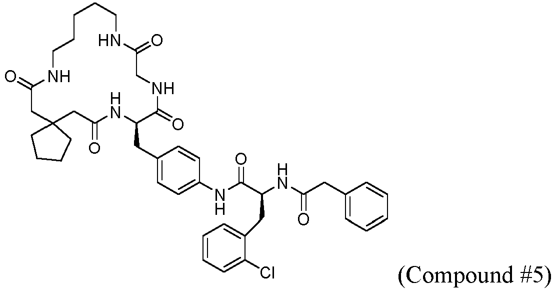 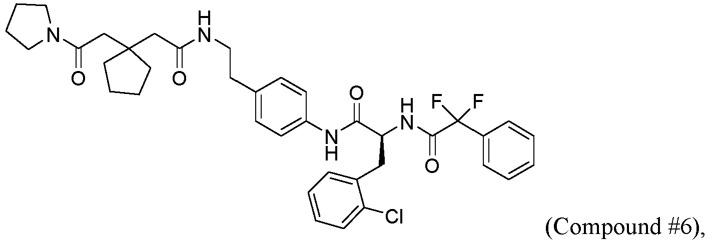 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

Yet further provided herein is a kit for determining an amount of modulator-bound IL-17 in a sample comprising IL-17 and a SM modulator of IL-17, the kit comprising:
i. a first capture antibody, wherein upon contact with the sample, the first capture antibody forms a first complex comprising the first capture antibody and the IL-17 unbound to the SM modulator, and a second complex comprising the first capture antibody and the IL-17 bound to the SM modulator;
ii. a first detection agent, wherein upon contact with the first complex and the second complex, the first detection agent, forms a third complex comprising the first capture antibody, the IL-17 unbound to the SM modulator, and the first detection agent, but not a fourth complex comprising the first capture antibody, the IL-17 bound to the SM modulator, and the first detection agent; and
iii. a second capture antibody, wherein upon contact with the sample, the second capture antibody forms a fifth complex comprising the second capture antibody and the IL-17 unbound to the SM modulator, and a sixth complex comprising the second capture antibody and the IL-17 bound to the SM modulator;
iv. a second detection agent, wherein upon contact with the fifth complex and the sixth complex, the second detection agent, forms a seventh complex comprising the second capture antibody, the IL-17 unbound to the SM modulator, and the second detection agent, and an eighth complex comprising the second capture antibody, the IL-17 bound to the SM modulator, and the second detection agent, respectively,
wherein the SM modulator is selected from the group consisting of Compounds #1-6 having the following structures and pharmaceutically acceptable salts thereof: 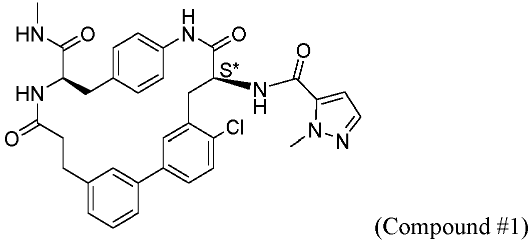 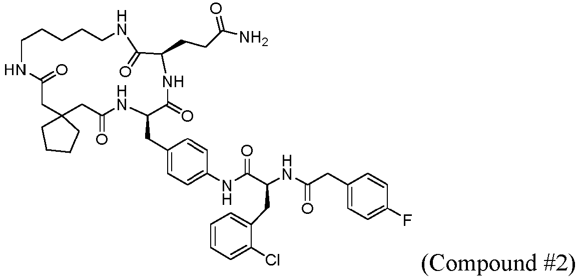 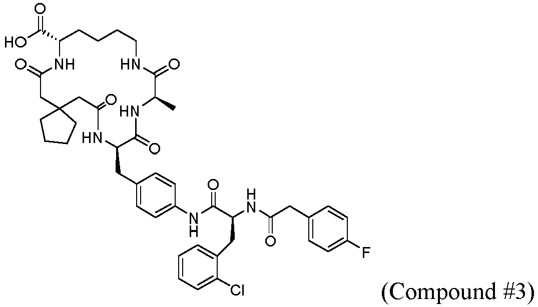 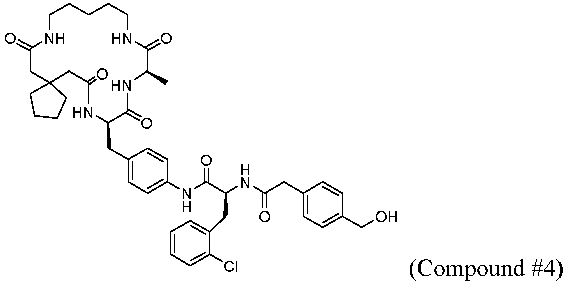 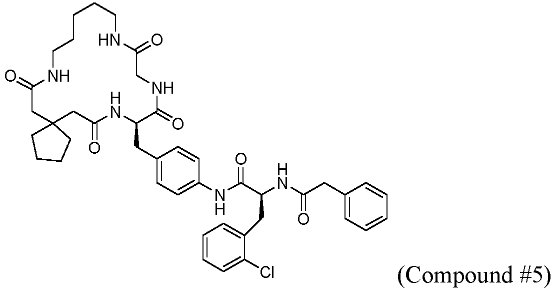 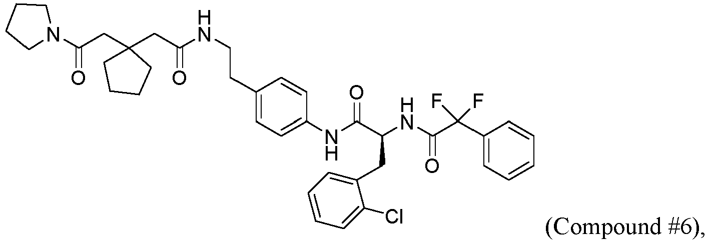 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agentIn one embodiment of any of the kits described above, the first detection agent and/or the second detection agent, is labeled with a detectable label, more preferably, the label is an enzyme or biotin.

In a yet further embodiment of any of the kits described above, the second capture antibody and the second detection agent are selected from the pairs in the following table:

| **Second capture antibody** | | **Second detection agent** | |
|---|---|---|---|
| **Company** | **CAT#** | **Company** | **CAT#** |
| R&D Systems, Inc. | Capture antibody of DuoSet ELISA kit DY317 | R&D Systems, Inc. | Detection antibody of DuoSet ELISA kit DY317 |
| R&D Systems, Inc. | Capture antibody of DuoSet ELISA kit DY317 | R&D Systems, Inc. | Detection antibody of human IL-17 HS ELISA Kit HS170 |
| R&D Systems, Inc. | Capture antibody of DuoSet ELISA kit DY317 | CovalAb R&D (Antibodies Online) | antibody ABIN1724556 |
| R&D Systems, Inc. | Capture antibody of DuoSet ELISA kit DY317 | Creative Diagnostics | Anti-IL 17A monoclonal antibody CABT-ZC1184 |
| R&D Systems, Inc. | Capture antibody of DuoSet ELISA kit DY317 | Mabtech AB | Anti-human IL-17 antibody 3520-3-250 |
| R&D Systems, Inc. | Capture antibody of DuoSet ELISA kit DY317 | MyBioSource (MBS) | Anti-IL-17A antibody MBS592444 |
| R&D Systems, Inc. | Capture antibody of DuoSet ELISA kit DY317 | MyBioSource (MBS) | Anti-IL-17A antibody MBS592430 |
| R&D Systems, Inc. | Capture antibody of DuoSet ELISA kit DY317 | MyBioSource (MBS) | Anti-IL-17A antibody MBS689609 |
| R&D Systems, Inc. | Capture antibody of DuoSet ELISA kit DY317 | MyBioSource (MBS) | Anti-IL-17A antibody MBS592432 |
| R&D Systems, Inc. | Capture antibody of DuoSet ELISA kit DY317 | NOVUS Biologicals LLC | IL-17/IL-17A antibody DDX0336B-100 |
| R&D Systems, Inc. | Capture antibody of DuoSet ELISA kit DY317 | Antibodies Online | ABIN786722 |
| R&D Systems, Inc. | Capture antibody of DuoSet ELISA kit DY317 | OriGene Technologies Inc. | DDX0334B-50 |
| R&D Systems, Inc. | Capture antibody of DuoSet ELISA kit DY317 | OriGene Technologies Inc. | DDX0335B-50 |
| R&D Systems, Inc. | Capture antibody of DuoSet ELISA kit DY317 | Rockland Immunochemicals Inc. | IL-17A biotin conjugated antibody 209-306-B32 |

In a yet further embodiment of any one of the kits described above, the second capture antibody and the second detection agent are capture antibody and detection antibody of DuoSet ELISA kit DY317 (from R&D Systems, Inc.), respectively.

Other aspects, features and advantages of the invention will be apparent from the following disclosure, including the detailed description of the invention and its preferred embodiments and the appended claims.

### BRIEF DESCRIPTION OF THE DRAWINGS

The foregoing summary, as well as the following detailed description of preferred embodiments of the present application, will be better understood when read in conjunction with the appended drawings. It should be understood, however, that the application is not limited to the precise embodiments shown in the drawings.

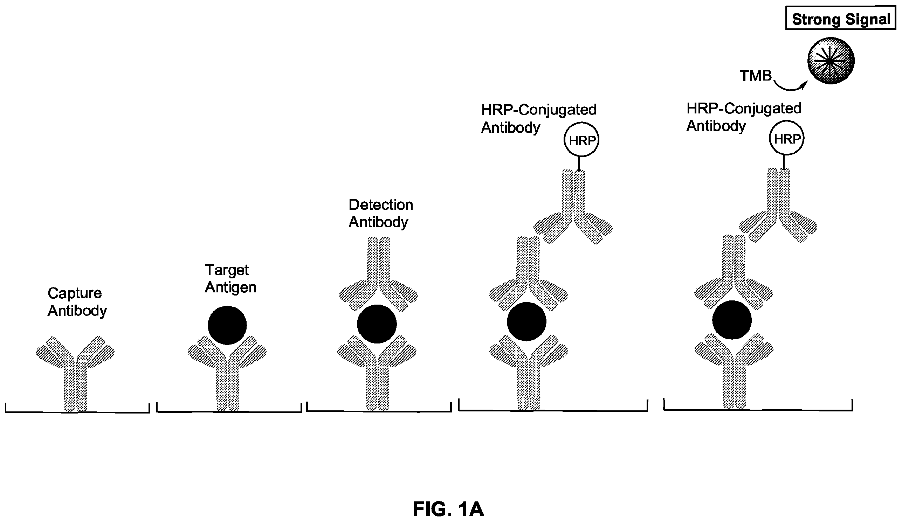

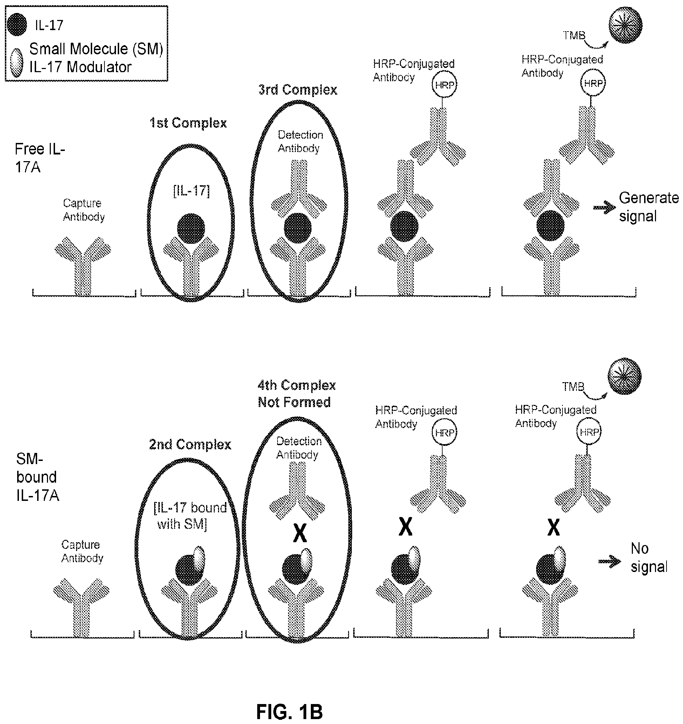

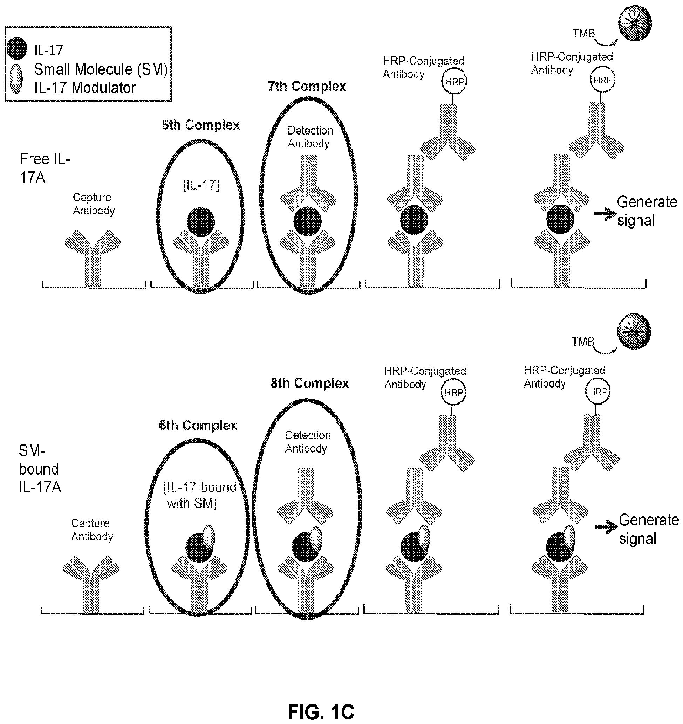

**FIGS. 1A-1C** show a schematic representation of an IL-17 target engagement assay: FIG. 1A - immunoassay to detect IL-17 using a capture antibody/detection agent pair; FIG. 1B - immunoassay to only detect free IL-17 (but not modulator-bound IL-17), in the presence of a small molecule (SM) modulator, using inventive capture antibody/detection agent pairs; and FIG. 1C - immunoassay to detect total IL-17 including both free IL-17 and modulator-bound IL-17 (i.e., IL-17 that are bound with SM modulator) using a currently available capture antibody/detection agent pair.

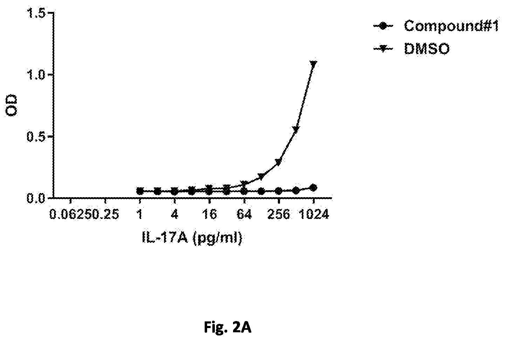

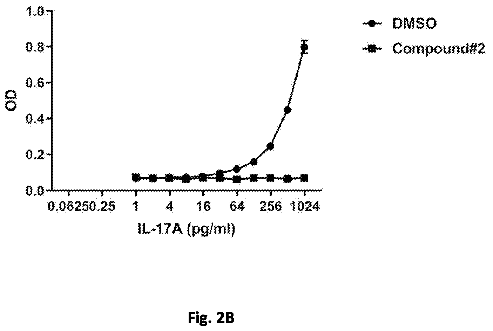

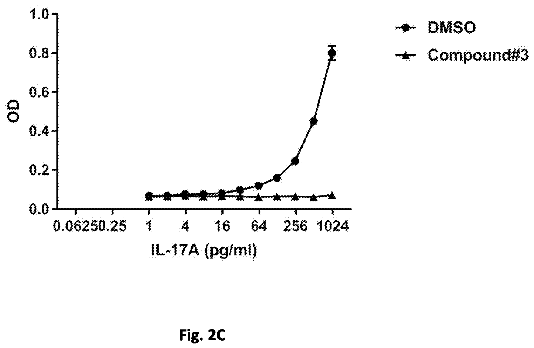

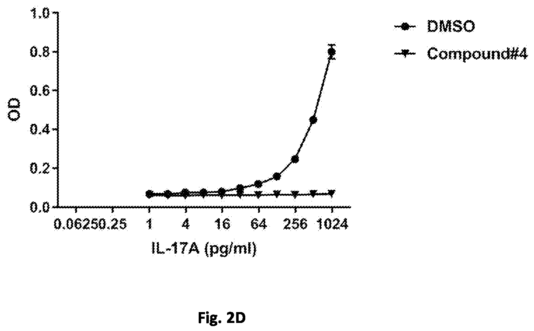

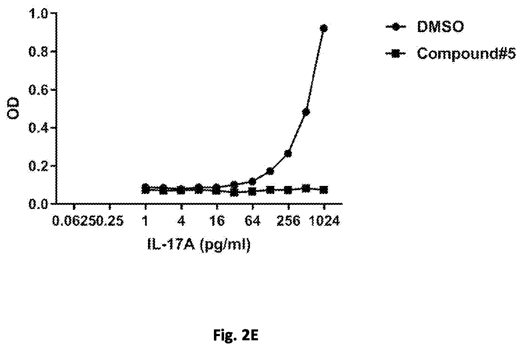

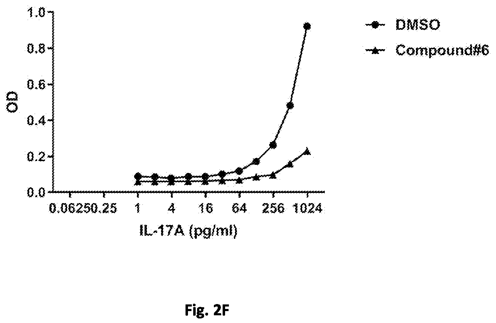

**FIGS. 2A-2F** shows detection of free IL-17, but not modulator-bound IL-17, using the capture antibody of DuoSet ELISA kit DY317 (R&D Systems, Inc.) pairing with mAb4538 (antibody of IL-17 from Janssen Biotech, Inc.) as the detection agent, in samples comprising small molecule (SM) modulators Compound #1, Compound #2, Compound #3, Compound #4, Compound #5, and Compound #6, respectively.

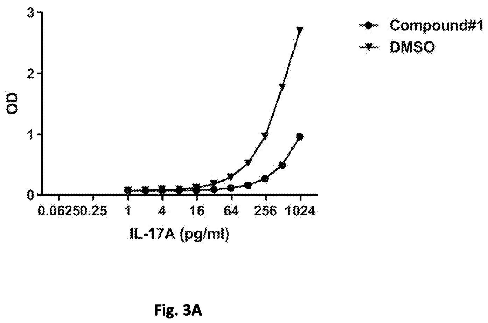

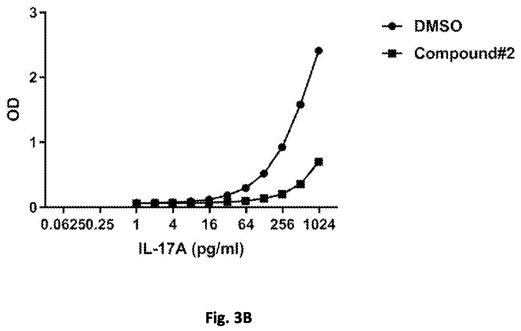

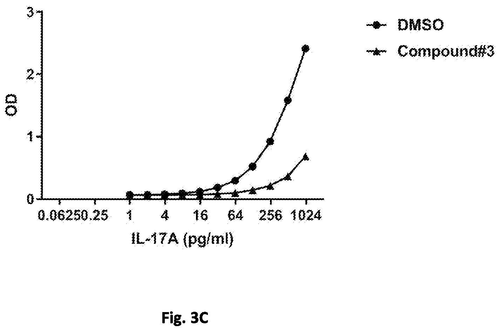

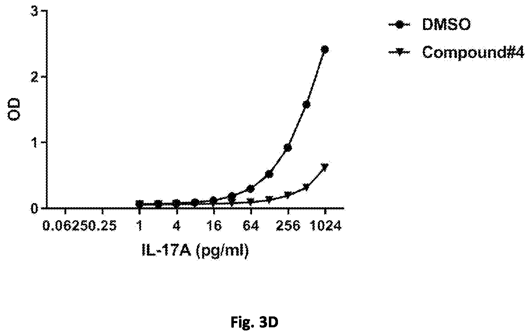

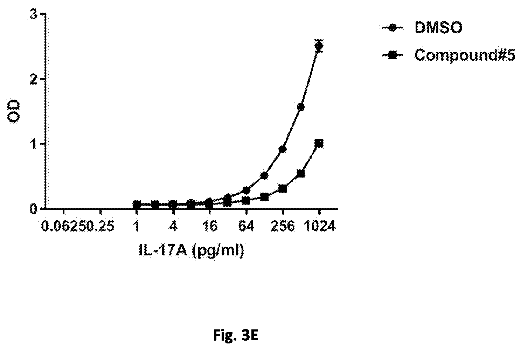

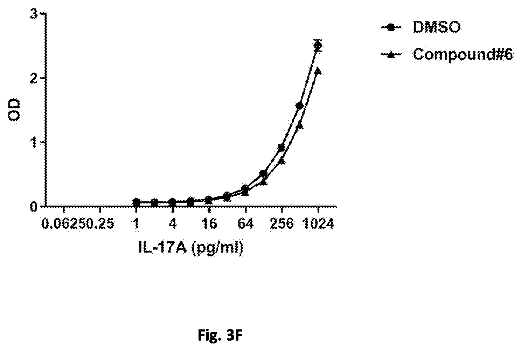

**FIGS. 3A-3F** shows detection of total IL-17 using DuoSet ELISA kit DY317 in samples comprising SM modulators Compound #1, Compound #2, Compound #3, Compound #4, Compound #5, and Compound #6, respectively.

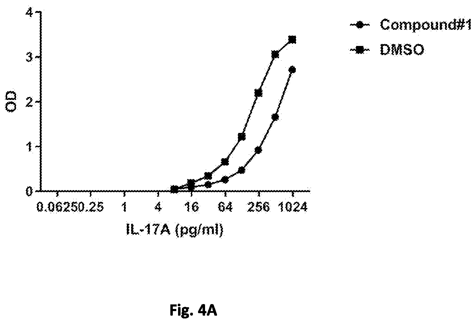

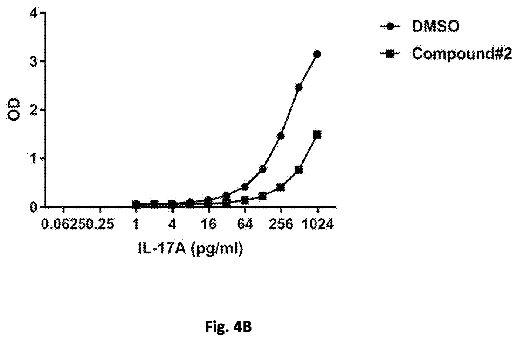

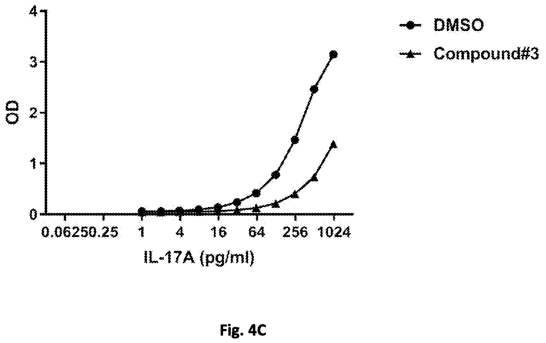

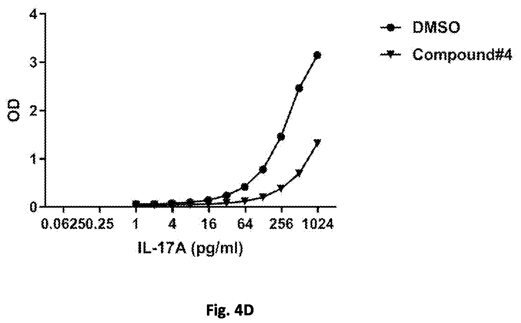

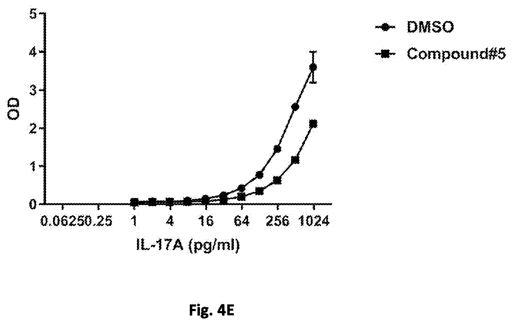

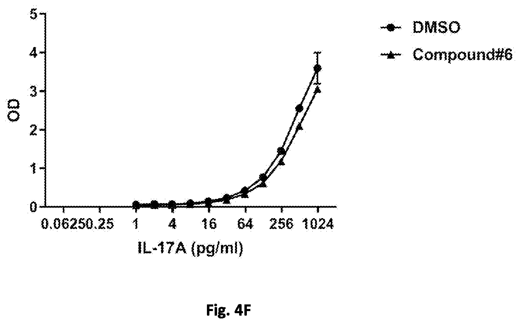

**FIGS. 4A-4F** shows detection of total IL-17 using the capture antibody of DuoSet ELISA kit DY317 pairing with antibody ABIN1724556 (from Covalab Antibodies Online) as the detection agent in samples comprising SM modulators Compound #1, Compound #2, Compound #3, Compound #4, Compound #5, and Compound #6, respectively.

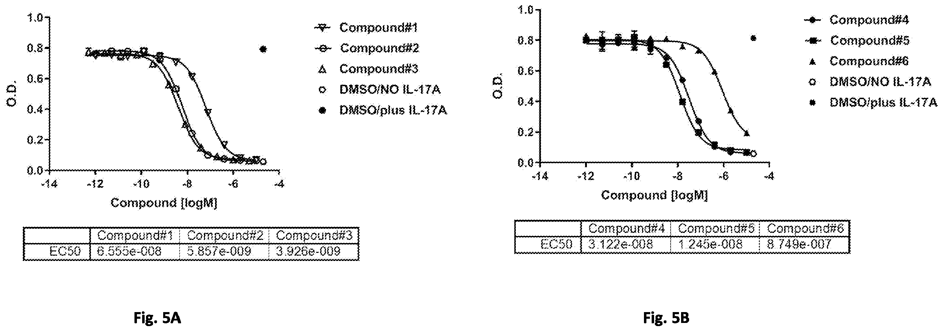

**FIGS. 5A-5B** shows detection of free IL-17, using the capture antibody of DuoSet ELISA kit DY317 pairing with mAb4538 as the detection agent, in samples comprising a range of concentrations of SM modulators.

### DETAILED DESCRIPTION

Various publications, articles and patents are cited or described in the background and throughout the specification. Discussion of documents, acts, materials, devices, articles or the like which has been included in the present specification is for the purpose of providing context for the invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim. Any of the aforementioned terms of "comprising", "containing", "including", and "having", whenever used herein in the context of an aspect or embodiment of the application can be replaced with the term "consisting of" or "consisting essentially of" to vary scopes of the disclosure.

As used herein, the conjunctive term "and/or" between multiple recited elements is understood as encompassing both individual and combined options. For instance, where two elements are conjoined by "and/or," a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Unless otherwise stated, any numerical value, such as a concentration or a concentration range described herein, are to be understood as being modified in all instances by the term "about." Thus, a numerical value typically includes ± 10% of the recited value. For example, a concentration of 1 mg/mL includes 0.9 mg/mL to 1.1 mg/mL. Likewise, a concentration range of 1 mg/mL to 10 mg/mL includes 0.9 mg/mL to 11 mg/mL. As used herein, the use of a numerical range expressly includes all possible subranges, all individual numerical values within that range, including integers within such ranges and fractions of the values unless the context clearly indicates otherwise.

The phrases "percent (%) sequence identity" or "% identity" or "% identical to" when used with reference to an amino acid sequence describe the number of matches ("hits") of identical amino acids of two or more aligned amino acid sequences as compared to the number of amino acid residues making up the overall length of the amino acid sequences. In other terms, using an alignment, for two or more sequences the percentage of amino acid residues that are the same (e.g. 90%, 91%, 92%, 93%, 94%, 95%, 97%, 98%, 99%, or 100% identity over the full-length of the amino acid sequences) may be determined, when the sequences are compared and aligned for maximum correspondence as measured using a sequence comparison algorithm as known in the art, or when manually aligned and visually inspected. The sequences which are compared to determine sequence identity may thus differ by substitution(s), addition(s) or deletion(s) of amino acids. Suitable programs for aligning protein sequences are known to the skilled person. The percentage sequence identity of protein sequences can, for example, be determined with programs such as CLUSTALW, Clustal Omega, FASTA or BLAST, e.g. using the NCBI BLAST algorithm (Altschul SF, et al (1997), Nucleic Acids Res. 25:3389-3402).

As used herein, "subject" means any animal, preferably a mammal, most preferably a human, who will be or has been treated by a method according to an embodiment of the application. The term "mammal" as used herein, encompasses any mammal. Examples of mammals include, but are not limited to, cows, horses, sheep, pigs, cats, dogs, mice, rats, rabbits, guinea pigs, non-human primates (NHPs) such as monkeys or apes, humans, etc., more preferably a human.

In an attempt to help the reader of the application, the description has been separated in various paragraphs or sections or is directed to various embodiments of the application. These separations should not be considered as disconnecting the substance of a paragraph or section or embodiments from the substance of another paragraph or section or embodiments. To the contrary, one skilled in the art will understand that the description has broad application and encompasses all the combinations of the various sections, paragraphs and sentences that can be contemplated. The discussion of any embodiment is meant only to be exemplary and is not intended to suggest that the scope of the disclosure, including the claims, is limited to these examples.

As used herein, "IL-17" or "IL-17A" refers to interleukin 17A. It is also named IL17, CTLA8, or CTLA-8. Interleukin 17A is a pro-inflammatory cytokine. This cytokine is produced by a type of T helper cell known as T helper 17 cells in response to their stimulation with IL-23. The protein encoded by IL17A is a founding member of the IL-17 family, including IL17A, IL-17B, IL-17C, IL-17D, IL-17E and IL-17F. IL-17E is also known as IL-25. All members of the IL-17 family have a similar protein structure. IL-17 regulates the activities of NF-kappaB and mitogen-activated protein kinases. It can stimulate the expression of IL-6 and cyclooxygenase-2 (PTGS2/COX-2), as well as enhance the production of nitric oxide (NO). High levels of IL17 are associated with several chronic inflammatory diseases including rheumatoid arthritis, psoriasis and multiple sclerosis. "IL-17" includes IL-17A from any animal species, as well as a recombinant product of IL-17A. IL-17 could be in homodimer of IL-17A or in heterodimer of IL-17A and IL-17F. An exemplary amino acid sequence of human IL-17 is represented in GenBank Accession No. NP_002181.1, which can be encoded by a nucleic acid sequence such as that of GenBank Accession No. NM_002190.3.

The term "modulator" as used herein refers to any agents or molecules that can bind to IL-17, including small molecule compounds and large molecules such as antibodies.

The terms "small molecule modulator(s)" and/or "SM modulator(s)" and/or "SM modulator(s) of IL-17" are used interchangeably herein and refer to any small molecule compound that can bind to IL-17 and has a molecular weight ranging from about 100 g/mol to about 1500 g/mol, or from 400 g/mol to about 1050 g/mol, or from 500 g/mol to about 1000 g/mol, or from about 600 g/mol to about 900 g/mol, or from about 300 g/mol to about 750 g/mol, or from about 500 to about 800 g/mol. Alternatively, the SM modulators may have a molecular weight ranging from a lower limit of about 100 g/mol or 200 g/mol or about 300 g/mol or about 400 g/mol or about 500 g/mol or about 600 g/mol to a upper limit of about 400 g/mol or about 500 g/mol or about 600 or about 700 g/mol or about 800 g/mol or about 900 g/mol or about 1000 g/mol or about 1100 g/mol, or about 1200 g/mol or about 1300 g/mol or about 1400 g/mol or about 1500 g/mol.

The terms "large molecule modulator(s)" and/or "LM modulator(s)" and/or "LM modulator(s) of IL-17" are used interchangeably herein and refer to any large molecule compound (e.g., antibodies, proteins, etc.) that can bind to IL-17 and has a molecular weight of above about 1500 daltons, or preferably has a molecular weight ranging from about 2000 daltons to about 250,000 daltons. The dalton (symbol Da) is used herein as a unit of molar mass, with the definition 1 Da = 1 g/mol

For small molecules, the absolute stereochemistry is specified according to the Cahn-Ingold-Prelog R-S system. Chiral centers, of which the absolute configurations are known, are depicted or labelled by prefixes R and S, assigned by the standard sequence-rule procedure, and preceded when necessary by the appropriate locants (Pure & Appl. Chem. 45, 1976, 11-30). Certain examples contain chemical structures that are depicted or labelled as an (R*) or (S*). When (R*) or (S*) is used in the name of a compound or in the chemical representation of the compound, it is intended to convey that the compound is a pure single isomer at that stereocenter; however, absolute configuration of that stereocenter has not been established. Thus, a compound designated as (R*) refers to a compound that is a pure single isomer at that stereocenter with an absolute configuration of either (R) or (S), and a compound designated as (S*) refers to a compound that is a pure single isomer at that stereocenter with an absolute configuration of either (R) or (S). For example, 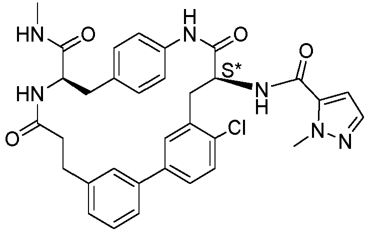 refers to a compound that is either: 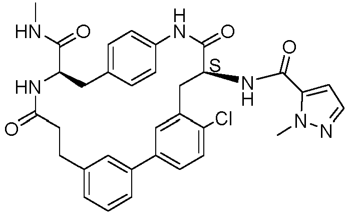 or

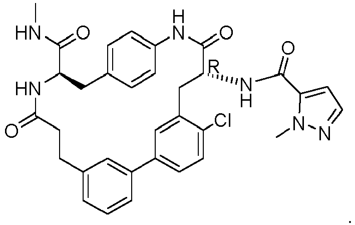

The term "free IL-17" as used herein refers to an IL-17 molecule(s) that is not bound to a small molecule modulator or a large molecule modulator in a biological sample.

The terms "sample" and "biological sample" are used interchangeably herein and encompass a variety of sample types obtained or derived from an organism and can be used in a diagnostic or monitoring assay. The terms encompass blood and other liquid samples of biological origin, solid tissue samples, such as a biopsy specimen or tissue cultures or cells derived therefrom and the progeny thereof. The terms encompass samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples.

As used herein, the term "antibody" is used in a broad sense and includes immunoglobulin or antibody molecules including human, humanized, composite and chimeric antibodies and antibody fragments that are monoclonal or polyclonal. In general, antibodies are proteins or peptide chains that exhibit binding specificity to a specific antigen. The antibody can be derived from any species and can be IgM, IgG (e.g. IgGl, IgG2, IgG3, or IgG4), IgD, IgA, or IgE, for example.

As used herein, the term "antigen-binding fragment" refers to an antibody fragment such as, for example, a diabody, a Fab, a Fab', a F(ab')2, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), a single domain antibody (sdab) an scFv dimer (bivalent diabody), a multispecific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure.

The term "monoclonal antibody" as used herein refers to a preparation of antibodies of single molecular composition.

The term "polyclonal antibody" as used herein refers to a composition of different antibody molecules that is capable of binding to or reacting with several different specific antigenic determinants on the same or on different antigens. The variability in antigen specificity of a polyclonal antibody is located in the variable regions of the individual antibodies constituting the polyclonal antibody, in particular in the complementarity determining regions CDRl, CDR2 and CDR3 regions.

The term "capture agent" as used herein refers to an agent that specifically binds to IL-17 regardless of whether the IL-17 is bound to a modulator or not. A "capture agent" is intended to encompass those agents, such as antibodies or soluble receptors, which specifically bind to an IL-17 unbound to a modulator and the IL-17 bound to the modulator. Such capture agents, such as capture antibodies, can be commercially available or investigational agents. Capture agents, such as capture antibodies, can be provided in solution or pre-coated to a surface.

The term "detection agent" as used herein refers to an agent that specifically binds to either IL-17 unbound to a modulator or the IL-17 bound to the modulator, or both. In a preferred embodiment, the term "detection agent" refers to an agent that specifically binds to an IL-17 unbound to a modulator but not the IL-17 bound to the modulator. A "detection agent," such as a detecting antibody, or a detecting soluble receptor, can be used to detect either an IL-17 unbound to a modulator or the IL-17 bound to the modulator. Detection agents, such as detection antibodies, can be commercially available or investigational. The detection agent can be detected by any method known in the art, such as using a radioactive or fluorescent tag, or by binding directly or indirectly to an enzyme or biotin. Preferably, the detection agent, such as detection antibody, is labeled with a detectable label, such as an enzyme or biotin.

The enzyme can convert certain substrates to detectable products. For example, the enzyme can be one of the following enzymes commonly used in enzyme-linked immunosorbent assays (ELISA):
- Horseradish Peroxide (HRP), often used for conjugation with a protein turns o-phenylenediamine dihydrochloride (OPD) into an amber product;
- HRP turns 3,3',5,5'-tetramethylbenzidine (TMB) into a blue product which becomes yellow in the presence of sulfuric or phosphoric acid;
- HRP turns 2,2'-Azinobis [3-ethylbenzothiazoline-6-sulfonic acid]-diammonium salt (ABTS) into a green product; and
- Alkaline phosphatase turns p-nitrophenyl phosphate (PNPP) into a yellow product.

Biotin is a water-soluble B vitamin, also called vitamin B7. Biotin can bind to Avidin, Streptavidin and NeutrAvidin with considerable affinity. These proteins (Avidin, Streptavidin and NeutrAvidin) can be further conjugated to the aforementioned detectable enzymes to produce signals.

The term "specifically binding" refers to binding between two agents, for example, an antigen and an antibody, characterized by the ability of one agent (e.g., antigen) to associate with another agent (e.g., antibody) even in the presence of many other diverse agents, i.e., to show preferential binding of one agent for another in a heterogeneous mixture of agents. The term "KD" refers to the dissociation constant, which is obtained from the ratio of Kd to Ka (i.e., Kd/Ka) and is expressed as a molar concentration (M). As used herein, an antibody that "specifically binds to" an antigen refers to an antibody or an antigen binding fragment thereof that binds to the antigen with a KD of 1×10⁻⁷ M or less, preferably 1×10⁻⁸ M or less, more preferably 5×10⁻⁹ M or less, 1×10⁻⁹ M or less, 5×10⁻¹⁰ M or less, or 1×10⁻¹⁰ M or less. KD values for a binding agent can be determined using methods in the art in view of the present disclosure. For example, the KD of an antibody can be determined by using surface plasmon resonance, such as by using a biosensor system, e.g., a Biacore^{®} system, or by using bio-layer interferometry technology, such as an Octet RED96 system. The smaller the value of the KD of an antibody, the higher affinity that the antibody binds to a target antigen.

The term "kit" as used herein refers to a combination of reagents and other materials. It is contemplated that the kit may include reagents such as buffering agents, protein stabilizing reagents, signal producing systems (e.g., florescence signal generating systems), antibodies, control proteins, as well as testing containers (e.g., microtiter plates, etc.). It is not intended that the term "kit" be limited to a particular combination of reagents and/or other materials. In one embodiment, the kit further comprises instructions for using the reagents. The test kit may be packaged in any suitable manner, typically with the elements in a single container or various containers as necessary along with a sheet of instructions for carrying out the test. In some embodiments, the kits also preferably include a positive control sample. Kits may be produced in a variety of ways known in the art.

### IL-17 Target Engagement Assay

The present application relates generally to an IL-17 target engagement assay. Using the present application to measure the amount of free IL-17 (that is, IL-17 not bound to SM modulator(s)) in a sample comprising the IL-17 and SM modulator(s), together with a total IL-17 detection assay, the level of IL-17 target engagement by SM modulator can be determined.

In studies involving SM modulators, currently available assays can only measure the total amount of IL-17 in a biological sample, since the detection antibody in the assay is unable to differentiate between free IL-17 (i.e., IL-17 not bound to a SM modulator(s)) and IL-17 bound to a SM modulator(s). Thus, the binding between IL-17 and a SM modulator(s) in such biological samples thereof cannot be monitored. This application, however, provides novel methods of determining the amount of free IL-17 in a sample in the presence of small modulators of IL-17. By subtracting the amount of free IL-17 from the total amount of IL-17, the level of IL-17 engagement by a SM modulator in a biological sample can be determined. As a result, this innovative method can be used to study clinically relevant parameters, such as pharmacokinetic (PK) and pharmacodynamic (PD) information, which in turn, can aid in the determination of an optimal dosage to maximize efficacy and safety. Moreover, the methods described herein can be applied to healthy subjects or patients because the innovative methods can measure free IL-17 in the presence of an SM modulator(s) independent of disease readout(s).

Accordingly, the present application provides a method of determining an amount of free IL-17 in a sample comprising IL-17 and SM modulator of IL-17, the method comprising (see Fig. 1B):
i. contacting the sample with a first capture antibody to form a mixture comprising a first complex comprising the first capture antibody and the IL-17 unbound to the SM modulator, and a second complex comprising the first capture antibody and the IL-17 bound to the SM modulator;
ii. contacting the mixture from step i) with a first detection agent (preferably a first detection antibody) to thereby form a third complex comprising the first capture antibody, the IL-17 unbound to the SM modulator, and the first detection agent, but not a fourth complex comprising the first capture antibody, the IL-17 bound to the SM modulator, and the first detection agent; and
iii. determining the amount of the free IL-17 by measuring the amount of the first detection agent in the third complex,
wherein the SM modulator is selected from the group consisting of Compounds #1-6 having the following structures and pharmaceutically acceptable salts thereof: 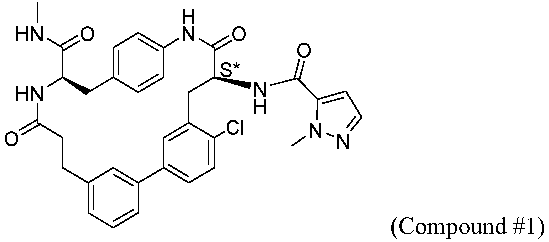 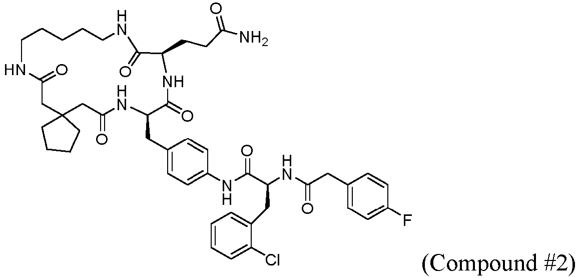 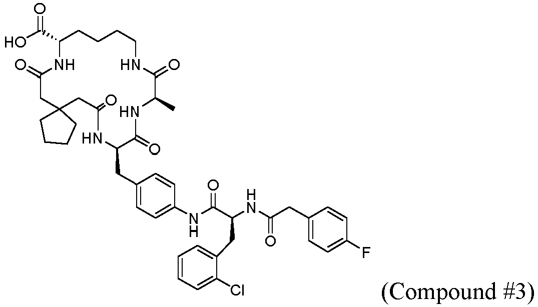 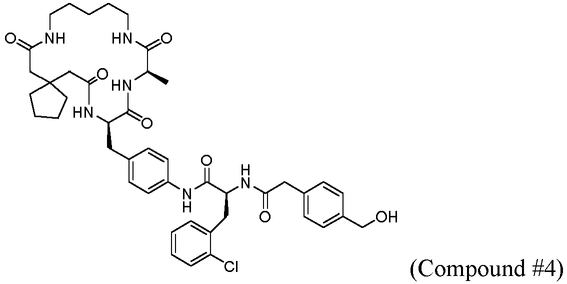 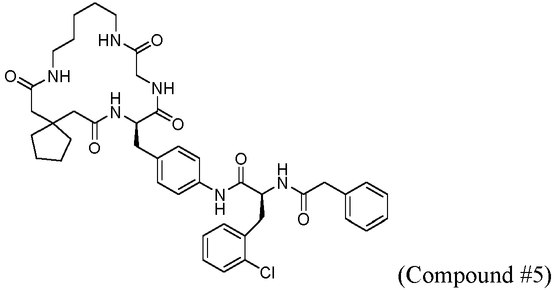 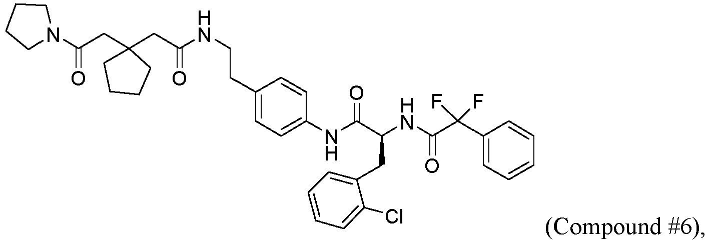 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

After contacting the sample with the first capture antibody, the first capture antibody specifically binds to the free IL-17 and the IL-17 bounded to the SM modulators in the sample to form the first and second complexes, respectively, in the mixture. In certain embodiments, the first capture antibody is attached to a solid carrier and the mixture contains the first and second IL-17 complexes attached to the solid carrier together with the rest of the sample.

As used herein, the term "first detection agent" refers to an agent which specifically binds to the free IL-17 that are captured by the first capture antibody, but not the modulator-bound IL-17 that are captured by the first capture antibody. In particular, when contacting the first detection agent with the first and second complexes, it forms a third complex comprising the first capture antibody, the free IL-17, and the first detection agent, but does not form a fourth complex comprising the first capture antibody, the modulator-bound IL-17, and the first detection agent.

Thus, the first capture antibody and the first detection agent are used in pairs to measure the amount of free IL-17 in a sample comprising IL-17 and SM modulator of IL-17.

Exemplary first detection agent and pairing first antibody of the disclosure are listed below in Table 1.

**Table 1. "First Detection Agent"/ "First Capture Antibody" Pairs for measuring free IL-17**

| **First capture antibody** | | **First detection agent** | |
|---|---|---|---|
| **Company** | **CAT# or ID** | **Company** | **CAT# or ID** |
| R&D Systems, Inc. | Capture antibody of DuoSet ELISA kit DY317 | Thermo Fisher Scientific, Inc. | Detection antibody of HS ELISA human IL-17A Kit BMS2017HS |
| R&D Systems, Inc. | Capture antibody of DuoSet ELISA kit DY317 | NOVUS Biologicals LLC | Detection antibody of HS ELISA human IL17A Kit #NBP1-82423 |
| Janssen Biotech, Inc. | mAb7024 | NOVUS Biologicals LLC | Detection antibody of HS ELISA human IL17A Kit #NBP1-82423 |
| R&D Systems, Inc. | Capture antibody of DuoSet ELISA kit DY317 | BioLegend, Inc. | Anti-human IL-17A antibody 512702 |
| R&D Systems, Inc. | Capture antibody of DuoSet ELISA kit DY317 | Janssen Biotech, Inc. | mAb3584 |
| Quanterix Corp. | Capture antibody of Il-17A Advantage Kit 101599 | Janssen Biotech, Inc. | mAb3584 |
| R&D Systems, Inc. | Capture antibody of DuoSet ELISA kit DY317 | Janssen Biotech, Inc. | mAb3077 |
| Quanterix Corp. | Capture antibody of Il-17A Advantage Kit 101599 | Janssen Biotech, Inc. | mAb3077 |
| R&D Systems, Inc. | Capture antibody of DuoSet ELISA kit DY317 | Janssen Biotech, Inc. | mAb732 |
| Quanterix Corp. | Capture antibody of Il-17A Advantage Kit 101599 | Janssen Biotech, Inc. | mAb732 |
| R&D Systems, Inc. | Capture antibody of DuoSet ELISA kit DY317 | Janssen Biotech, Inc. | mAb7024 |
| R&D Systems, Inc. | Capture antibody of DuoSet ELISA kit DY317 | Janssen Biotech, Inc. | mAb4538 |
| R&D Systems, Inc. | Capture antibody of DuoSet ELISA kit DY317 | Janssen Biotech, Inc. | mAb5548 |
| R&D Systems, Inc. | Capture antibody of DuoSet ELISA kit DY317 | Creative Diagnostics, Inc. | Anti-IL 17A monoclonal antibody DCABH-4543 |
| R&D Systems, Inc. | Capture antibody of DuoSet ELISA kit DY317 | Creative Diagnostics, Inc. | Anti-IL 17A monoclonal antibody CABT-ZC1183 |
| R&D Systems, Inc. | Capture antibody of DuoSet ELISA kit DY317 | Creative Diagnostics, Inc. | Anti-IL 17A monoclonal antibody CABT-WN1669 |
| R&D Systems, Inc. | Capture antibody of DuoSet ELISA kit DY317 | Invitrogen | IL-17A antibody MA 1069B |
| Quanterix Corp. | Capture antibody of Il-17A Advantage Kit 101599 | Invitrogen | IL-17A antibody MA 1069B |
| Janssen Biotech, Inc., | mAb7024 | Invitrogen | IL-17A antibody MA 1069B |
| R&D Systems, Inc. | Capture antibody of DuoSet ELISA kit DY317 | Mabtech AB | Anti-human IL-17A antibody 3520-5N-500 |
| Janssen Biotech, Inc. | mAb7024 | Mabtech AB | Anti-human IL-17A antibody 3520-5N-500 |
| R&D Systems, Inc. | Capture antibody of DuoSet ELISA kit DY317 | Sino Biological Inc. | Antibody ABIN5507411 |
| R&D Systems, Inc. | Capture antibody of DuoSet ELISA kit DY317 | R&D Systems, Inc. | Recombinant Human IL-17RA/IL-17R Fc Chimera Protein, CF, 177-IR-100 |
| R&D Systems, Inc. | Capture antibody of DuoSet ELISA kit DY317 | Sino Biological Inc. | IL-17RA protein 10895-H08H-B |

Among the first detection agents listed in Table 1, IL-17 antibodies mAb7024, mAb3584, mAb3077, mAb732, mAb4538, and mAb5548 from Janssen Biotech, Inc. are disclosed in U.S. Patent. No. 8,519,107. Detailed sequence information thereof is listed in Table 2.

**Table 2. Antibody Sequence**

| Antibody ID | Sequence |
|---|---|
| mAb4538 | LCDR1 - SEQ ID NO: 1 |
| | RASQNVWAFYLA |
| | LCDR2 - SEQ ID NO: 2 |
| | GASNRAT |
| | LCDR3 - SEQ ID NO: 3 |
| | QQYRSTLSLT |
| | HCDR1 - SEQ ID NO: 4 |
| | SSSAAWG |
| | HCDR2 - SEQ ID NO: 5 |
| | RISYRSKWYNDYAVSVKS |
| | HCDR3 - SEQ ID NO: 6 |
| | EVDSMYYSYFDI |
| | VL - SEQ ID NO: 7 EIVLTQSPATLSLSPGERATLSCRASQNVWAFYLAWYQQKPGQ APRLLIYGASNRATGIPARFSGSGSGTDFTLTISSLEPEDFAVYY CQQYRSTLSLTFGQGTKVEIK |
| | VH - SEQ ID NO: 8 QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSS |
| | LC - SEQ ID NO: 9 EIVLTQSPATLSLSPGERATLSCRASQNVWAFYLAWYQQKPGQ APRLLIYGASNRATGIPARFSGSGSGTDFTLTISSLEPEDFAVYY CQQYRSTLSLTFGQGTKVEIKGQPKAAPSVTLFPPSSEELQANK ATLVCLISDFYPGAVTVAWKADSSPVKAGVETTTPSKQSNNKY AASSYLSLTPEQWKSHRSYSCQVTHEGSTVEKTVAPTECS |
| | HC - SEQ ID NO: 10 QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |
| mAb732 | LCDR1 - SEQ ID NO: 1 |
| | RASQNVWAFYLA |
| | LCDR2 - SEQ ID NO: 2 |
| | GASNRAT |
| | LCDR3 - SEQ ID NO: 11 |
| | TQYYSSPSLT |
| | HCDR1 - SEQ ID NO: 4 |
| | SSSAAWG |
| | HCDR2 - SEQ ID NO: 5 |
| | RISYRSKWYNDYAVSVKS |
| | HCDR3 - SEQ ID NO: 6 |
| | EVDSMYYSYFDI |
| | VL - SEQ ID NO: 12 EIVLTQSPATLSLSPGERATLSCRASQNVWAFYLAWYQQKPGQ APRLLIYGASNRATGIPARFSGSGSGTDFTLTISSLEPEDFAVYY CTQYYSSPSLTFGQGTKVEIK |
| | VH - SEQ ID NO: 8 QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSS |
| | LC - SEQ ID NO: 33 EIVLTQSPATLSLSPGERATLSCRASQNVWAFYLAWYQQKPGQ APRLLIYGASNRATGIPARFSGSGSGTDFTLTISSLEPEDFAVYY CTQYYSSPSLTFGQGTKVEIKGQPKAAPSVTLFPPSSEELQANK ATLVCLISDFYPGAVTVAWKADSSPVKAGVETTTPSKQSNNKY AASSYLSLTPEQWKSHRSYSCQVTHEGSTVEKTVAPTECS |
| | HC - SEQ ID NO: 10 QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSMYYSYFDIWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT |
| | PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |
| mAb7024 | LCDR1 - SEQ ID NO: 13 |
| | RASQGISNWLN |
| | LCDR2 - SEQ ID NO: 14 |
| | AASSLQS |
| | LCDR3 - SEQ ID NO: 15 |
| | QQYSDDPT |
| | HCDR1 - SEQ ID NO: 16 |
| | SYAIS |
| | HCDR2 - SEQ ID NO: 17 |
| | SIIPWFGTTNYAQKFQG |
| | HCDR3 - SEQ ID NO: 18 |
| | DSEYYFDH |
| | VL - SEQ ID NO: 19 DIQMTQSPSSVSASVGDRVTITCRASQGISNWLNWYQQKPGKA PKLLIYAASSLQSGVPSRFSGSGSGTDFTLTISSLQPEDFATYYC QQYSDDPTFGQGTKVEIK |
| | VH - SEQ ID NO: 20 QVQLVQSGAEVKKPGSSVKVSCKASGGTFSSYAISWVRQAPGQ GLEWMGSIIPWFGTTNYAQKFQGRVTITADESTSTAYMELSSLR SEDTAVYYCARDSEYYFDHWGQGTLVTVSS |
| | LC - SEQ ID NO: 21 DIQMTQSPSSVSASVGDRVTITCRASQGISNWLNWYQQKPGKA PKLLIYAASSLQSGVPSRFSGSGSGTDFTLTISSLQPEDFATYYC QQYSDDPTFGQGTKVEIKGQPKAAPSVTLFPPSSEELQANKATL |
| | VCLISDFYPGAVTVAWKADSSPVKAGVETTTPSKQSNNKYAAS SYLSLTPEQWKSHRSYSCQVTHEGSTVEKTVAPTECS |
| | HC - SEQ ID NO: 34 QVQLVQSGAEVKKPGSSVKVSCKASGGTFSSYAISWVRQAPGQ GLEWMGSIIPWFGTTNYAQKFQGRVTITADESTSTAYMELSSLR SEDTAVYYCARDSEYYFDHWGQGTLVTVSSASTKGPSVFPLAP SSKSTSGGTAALGCLVKDYFPEPVTVSWNSGALTSGVHTFPAV LQSSGLYSLSSVVTVPSSSLGTQTYICNVNHKPSNTKVDKKVEP KSCDKTHTCPPCPAPELLGGPSVFLFPPKPKDTLMISRTPEVTCV VVDVSHEDPEVKFNWYVDGVEVHNAKTKPREEQYNSTYRVV SVLTVLHQDWLNGKEYKCKVSNKALPAPIEKTISKAKGQPREP QVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |
| mAb3584 | LCDR1 - SEQ ID NO: 1 |
| | RASQNVWAFYLA |
| | LCDR2 - SEQ ID NO: 2 |
| | GASNRAT |
| | LCDR3 - SEQ ID NO: 3 |
| | QQYRSTLSLT |
| | HCDR1 - SEQ ID NO: 4 |
| | SSSAAWG |
| | HCDR2 - SEQ ID NO: 5 |
| | RISYRSKWYNDYAVSVKS |
| | HCDR3 - SEQ ID NO: 35 |
| | EVDSIYYSYFDI |
| | VL - SEQ ID NO: 7 EIVLTQSPATLSLSPGERATLSCRASQNVWAFYLAWYQQKPGQ APRLLIYGASNRATGIPARFSGSGSGTDFTLTISSLEPEDFAVYY CQQYRSTLSLTFGQGTKVEIK |
| | VH - SEQ ID NO: 36 QVQLQQSGPGLVKPSQTLSLTCAISGDSVSSSSAAWGWIRQSPR GLEWLGRISYRSKWYNDYAVSVKSRITINPDTSKNQFSLQLNSV TPEDTAVYYCAREVDSIYYSYFDIWGQGTLVTVSS |
| | LC - SEQ ID NO: 9 EIVLTQSPATLSLSPGERATLSCRASQNVWAFYLAWYQQKPGQ APRLLIYGASNRATGIPARFSGSGSGTDFTLTISSLEPEDFAVYY CQQYRSTLSLTFGQGTKVEIKGQPKAAPSVTLFPPSSEELQANK ATLVCLISDFYPGAVTVAWKADSSPVKAGVETTTPSKQSNNKY AASSYLSLTPEQWKSHRSYSCQVTHEGSTVEKTVAPTECS |
| | HC - SEQ ID NO: 37 QVQLQQSGPGLVKPSQTLSLTCAISGDSVSSSSAAWGWIRQSPS RGLEWLGRISYRSKWYNDYAVSVKSRITINPDTSKNQFSLQLNS VTPEDTAVYYCAREVDSIYYSYFDIWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSRDELTKNQVSLTCLVKGFYPSDIAVEWESNG QPENNYKTTPPVLDSDGSFFLYSKLTVDKSRWQQGNVFSCSVM HEALHNHYTQKSLSLSPGK |
| mAb3077 | LCDR1 - SEQ ID NO: 13 |
| | RASQGISNWLN |
| | LCDR2 - SEQ ID NO: 14 |
| | AASSLQS |
| | LCDR3 - SEQ ID NO: 15 |
| | QQYSDDPT |
| | HCDR1 - SEQ ID NO: 16 |
| | SYAIS |
| | HCDR2 - SEQ ID NO: 38 |
| | SIIPWFGWTNYAQKFQG |
| | HCDR3 - SEQ ID NO: 18 |
| | DSEYYFDH |
| | VL - SEQ ID NO: 19 DIQMTQSPSSVSASVGDRVTITCRASQGISNWLNWYQQKPGKA PKLLIYAASSLQSGVPSRFSGSGSGTDFTLTISSLQPEDFATYYC QQYSDDPTFGQGTKVEIK |
| | VH - SEQ ID NO: 39 QVQLVQSGAEVKKPGSSVKVSCKASGGTFSSYAISWVRQAPGQ GLEWMGSIIPWFGWTNYAQKFQGRVTITADESTSTAYMELSSL RSEDTAVYYCARDSEYYFDHWGQGTLVTVSS |
| | LC - SEQ ID NO: 21 DIQMTQSPSSVSASVGDRVTITCRASQGISNWLNWYQQKPGKA PKLLIYAASSLQSGVPSRFSGSGSGTDFTLTISSLQPEDFATYYC QQYSDDPTFGQGTKVEIKGQPKAAPSVTLFPPSSEELQANKATL VCLISDFYPGAVTVAWKADSSPVKAGVETTTPSKQSNNKYAAS SYLSLTPEQWKSHRSYSCQVTHEGSTVEKTVAPTECS |
| | HC - SEQ ID NO: 22 QVQLVQSGAEVKKPGSSVKVSCKASGGTFSSYAISWVRQAPGQ GLEWMGSIIPWFGWTNYAQKFQGRVTITADESTSTAYMELSSL RSEDTAVYYCARDSEYYFDHWGQGTLVTVSSASTKGPSVFPLA PSSKSTSGGTAALGCLVKDYFPEPVTVSWNSGALTSGVHTFPA VLQSSGLYSLSSVVTVPSSSLGTQTYICNVNHKPSNTKVDKKVE PKSCDKTHTCPPCPAPELLGGPSVFLFPPKPKDTLMISRTPEVTC VVVDVSHEDPEVKFNWYVDGVEVHNAKTKPREEQYNSTYRV VSVLTVLHQDWLNGKEYKCKVSNKALPAPIEKTISKAKGQPRE PQVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |
| mAb5548 | LCDR1 - SEQ ID NO: 23 |
| | SGDNLGDKYAN |
| | LCDR2 - SEQ ID NO: 24 |
| | DDIDRPS |
| | LCDR3 - SEQ ID NO: 25 |
| | GSYDFFLGLIV |
| | HCDR1 - SEQ ID NO: 26 |
| | SYAMS |
| | HCDR2 - SEQ ID NO: 27 |
| | TISLTSGFTYYADSVKG |
| | HCDR3 - SEQ ID NO: 28 |
| | QLTLDV |
| | VL - SEQ ID NO: 29 QSVLTQPPSVSVAPGQTARISCSGDNLGDKYANWYQQKPGQA PVLVIYDDIDRPSGIPERFSGSNSGNTATLTISGTQAEDEADYYC GSYDFFLGLIVFGGGTKLTVL |
| | VH - SEQ ID NO: 30 QVQLLESGGGLVQPGGSLRLSCAASGFTFSSYAMSWVRQAPG KGLEWVSTISLTSGFTYYADSVKGRFTISRDNSKNTLYLQMNSL RAEDTAVYYCARQLTLDVWGQGTLVTVSS |
| | LC - SEQ ID NO: 31 QSVLTQPPSVSVAPGQTARISCSGDNLGDKYANWYQQKPGQA PVLVIYDDIDRPSGIPERFSGSNSGNTATLTISGTQAEDEADYYC GSYDFFLGLIVFGGGTKLTVLGQPKAAPSVTLFPPSSEELQANK ATLVCLISDFYPGAVTVAWKADSSPVKAGVETTTPSKQSNNKY AASSYLSLTPEQWKSHRSYSCQVTHEGSTVEKTVAPTECS |
| | HC - SEQ ID NO: 32 QVQLLESGGGLVQPGGSLRLSCAASGFTFSSYAMSWVRQAPG KGLEWVSTISLTSGFTYYADSVKGRFTISRDNSKNTLYLQMNSL RAEDTAVYYCARQLTLDVWGQGTLVTVSSASTKGPSVFPLAPS |
| | SKSTSGGTAALGCLVKDYFPEPVTVSWNSGALTSGVHTFPAVL QSSGLYSLSSVVTVPSSSLGTQTYICNVNHKPSNTKVDKKVEPK SCDKTHTCPPCPAPELLGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREPQ VYTLPPSRDELTKNQVSLTCLVKGFYPSDIAVEWESNGQPENN YKTTPPVLDSDGSFFLYSKLTVDKSRWQQGNVFSCSVMHEALH NHYTQKSLSLSPGK |

In certain embodiments, the first detection agent, such as first detection antibody, is labeled with a detectable label, preferably, the label is an enzyme or biotin. In further embodiment, the first detection agent is labeled with a marker such as biotin for interacting with a labelled enzyme such as streptavidin conjugated horse radish peroxidase, which works on tetramethylbenzidine in the presence of hydrogen peroxide to generate color signals for determination the amount of first detection agent.

As used herein, the terms "wash", "wash step", or "washing" refer to a process used to separate the total IL-17 (including both free IL-17 and modulator-bound IL-17), which are unbound to the capture antibody, from the mixture in step (i). The solution used for washing is generally a buffer ("washing buffer"). In some embodiments, the washing buffer contains low concentrations of detergents. In some embodiments, the washing buffer is a 10 mM phosphate buffer at a pH of about 7.4 comprising 150 mM NaCl and 0.05% Tween 20. The pH of the washing buffer is preferably in a range of about 6 to about 9. In some embodiments the pH is about 7.0. The wash step may be performed once or multiple times (e.g., one time, two times, three times, four times, five times, or six times). In some embodiments, the wash step is performed three to six times.

In certain embodiments, a wash step is performed after step (i) and before step (ii) of contact the mixture with the second detection age.

In some embodiments, no wash step is performed. For example, in some embodiments, no wash step is performed after step (i) and before step (ii) of contact the mixture with the second detection age.
The present application further provides methods of determining the target engagement of a SM modulator(s) of IL-17 in a subject, wherein the SM modulator is selected from the group consisting of Compounds #1-6 having the following structures and pharmaceutically acceptable salts thereof: 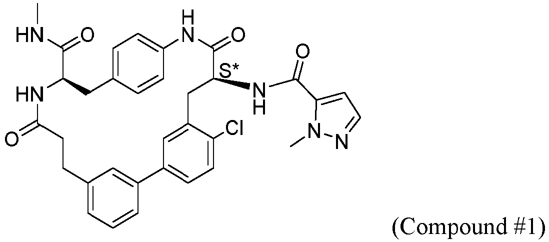 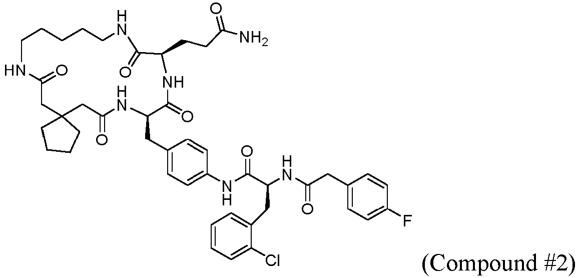 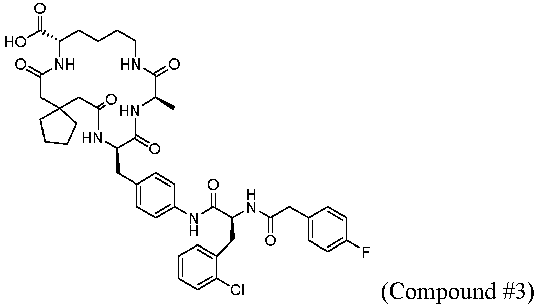 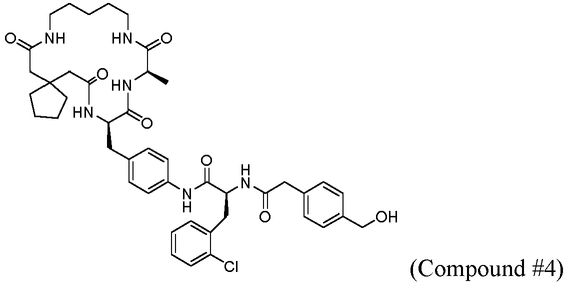 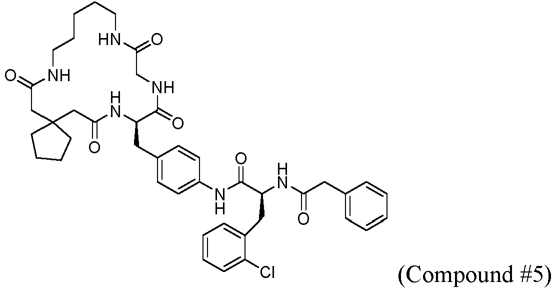 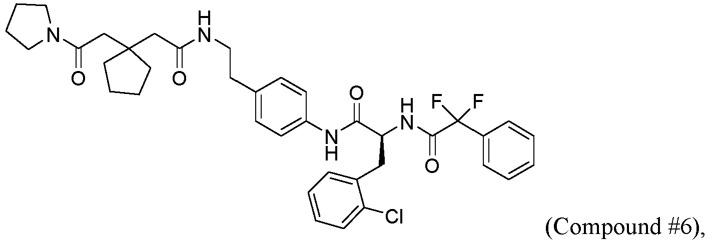 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent. The method comprises: a) determining the amount of free IL-17 in a sample from the subject before the administration of the SM modulator as described above; b) determining the amount of free IL-17 in a sample from the subject after the administration of the SM modulator as described above; and c) comparing the amounts of free IL-17 determined from steps a) and b). Or, the method may comprise (aa) determining the amount of free IL-17 in a sample from the subject at two or more time points after the administration of the SM modulator as described above; and (bb) evaluating changes of the amounts of free IL-17 at various time points after the administration of the SM modulator. As such, the target engagement activity of the SM modulators of IL-17 can be assessed and used to assist the adjustment of therapeutic regime of the SM modulators of IL-17.
The present application yet further provides methods of determining an amount of total IL-17 (free IL-17 and modulator-bound IL-17) in a sample comprising IL-17 and a SM modulator of IL-17 wherein the SM modulator is selected from the group consisting of Compounds #1-6 having the following structures and pharmaceutically acceptable salts thereof: 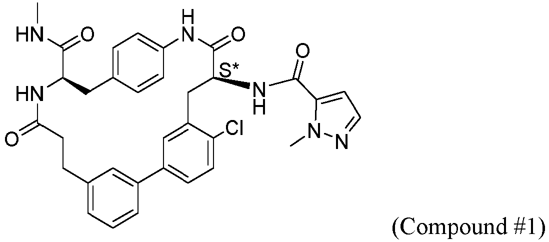 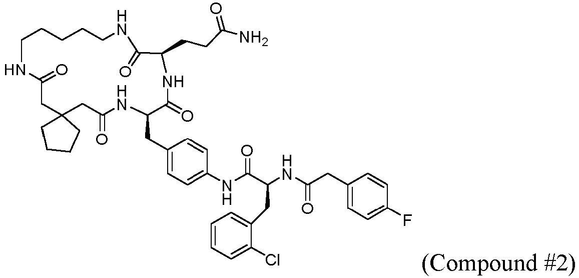 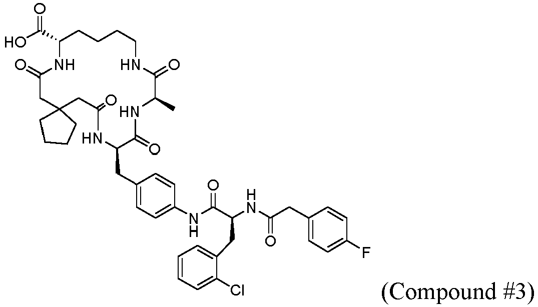 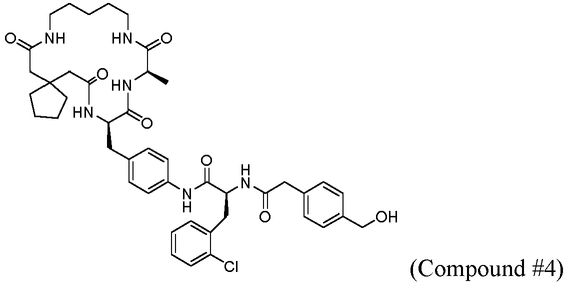 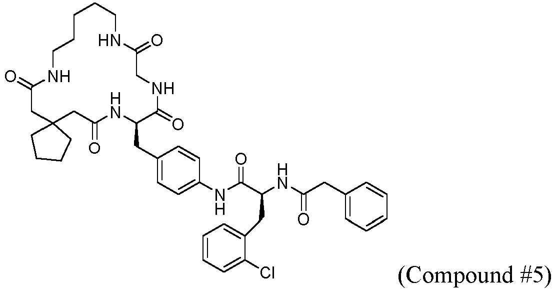 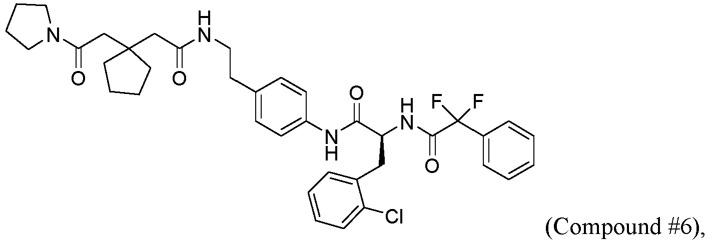 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent. The methods comprise (see *e.g.,* Fig. 1C):
i. contacting a portion of the sample with a second capture antibody to form a second mixture comprising a fifth complex comprising the second capture antibody and the IL-17 unbound to the SM modulator, and a sixth complex comprising the second capture antibody and the IL-17 bound to the SM modulator;
ii. contacting the second mixture with a second detection agent (preferably a second detection antibody) to thereby form a seventh complex comprising the second capture antibody, the IL-17 unbound to the SM modulator, and the second detection agent and an eighth complex comprising the second capture antibody, the IL-17 bound to the SM modulator, and the second detection agent;
iii. determining a total amount of the IL-17 by measuring the total amount of the second detection agent in the seventh complex and the eighth complex.

As used herein, the "second capture antibody" and the "second detection agent" (preferably "second detection antibody") are used in pairs to measure the total amount IL-17 (that is, free IL-17 and modulator-bound IL-17) in a sample. In particular, the second detection agent binds to both the free IL-17 that are captured by the second capture antibody and the modulator-bound IL-17 that are captured by the second capture antibody. Thus, when contacting the second detection agent with the second complex, it forms a seventh complex comprising the second capture antibody, the IL-17 unbound to the SM modulator and the second detection agent, and an eighth complex comprising the second capture antibody, the IL-17 bound to the SM modulator and the second detection agent.

Thus, the second capture antibody and the second detection agent are used in pairs to measure the amount of total IL-17 in a sample.

Exemplary second detection agent and pairing second antibody may be selected from the pairs listed below in Table 3.

**TABLE 3. "Second Detection Agent"/ "Second Capture Antibody" Pairs for measuring total IL-17**

| **Second capture antibody** | | **Second detection agent** | |
|---|---|---|---|
| **Company** | **CAT#** | **Company** | **CAT#** |
| R&D Systems, Inc. | Capture antibody of DuoSet ELISA kit DY317 | R&D Systems, Inc. | Detection antibody of DuoSet ELISA kit DY317 |
| R&D Systems, Inc. | Capture antibody of DuoSet ELISA kit DY317 | R&D Systems, Inc. | Detection antibody of human IL-17 HS ELISA Kit HS170 |
| R&D Systems, Inc. | Capture antibody of DuoSet ELISA kit DY317 | CovalAb R&D (Antibodies Online) | antibody ABIN1724556 |
| R&D Systems, Inc. | Capture antibody of DuoSet ELISA kit DY317 | Creative Diagnostics | Anti-IL 17A monoclonal antibody CABT-ZC1184 |
| R&D Systems, Inc. | Capture antibody of DuoSet ELISA kit DY317 | Mabtech AB | Anti-human IL-17 antibody 3520-3-250 |
| R&D Systems, Inc. | Capture antibody of DuoSet ELISA kit DY317 | MyBioSource (MBS) | Anti-IL-17A antibody MBS592444 |
| R&D Systems, Inc. | Capture antibody of DuoSet ELISA kit DY317 | MyBioSource (MBS) | Anti-IL-17A antibody MBS592430 |
| R&D Systems, Inc. | Capture antibody of DuoSet ELISA kit DY317 | MyBioSource (MBS) | Anti-IL-17A antibody MBS689609 |
| R&D Systems, Inc. | Capture antibody of DuoSet ELISA kit DY317 | MyBioSource (MBS) | Anti-IL-17A antibody MBS592432 |
| R&D Systems, Inc. | Capture antibody of DuoSet ELISA kit DY317 | NOVUS Biologicals LLC | IL-17/IL-17A antibody DDX0336B-100 |
| R&D Systems, Inc. | Capture antibody of DuoSet ELISA kit DY317 | Antibodies Online | ABIN786722 |
| R&D Systems, Inc. | Capture antibody of DuoSet ELISA kit DY317 | OriGene Technologies Inc. | DDX0334B-50 |
| R&D Systems, Inc. | Capture antibody of DuoSet ELISA kit DY317 | OriGene Technologies Inc. | DDX0335B-50 |
| R&D Systems, Inc. | Capture antibody of DuoSet ELISA kit DY317 | Rockland Immunochemicals Inc. | IL-17A biotin conjugated antibody 209-306-B32 |

In certain embodiments, the second detection agent is labeled with a detectable label, preferably, the label is an enzyme or biotin. In further embodiment, the second detection agent is labeled with a marker such as biotin for interacting with a labelled enzyme such as streptavidin conjugated horse radish peroxidase, which works on tetramethylbenzidine in the presence of hydrogen peroxide to generate color signals for determination the amount of second detection agent, such as second detection antibody.

In certain embodiments, a wash step is performed after step (i) and before step (ii) of contact the second mixture with the second detection agent.

In some embodiments, no wash step is performed. For example, in some embodiments, no wash step is performed after step (i) and before step (ii) of contact the second mixture with the second detection age.
The present application yet further provides methods of determining an amount of modulator-bound IL-17 in a sample comprising IL-17 and a SM modulator of IL-17 wherein the SM modulator is selected from the group consisting of Compounds #1-6 having the following structures and pharmaceutically acceptable salts thereof: 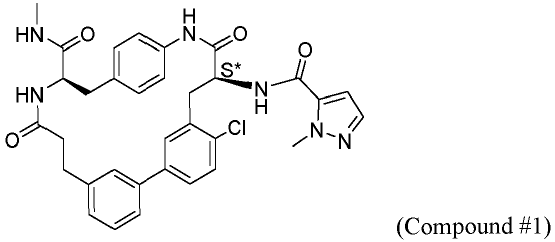 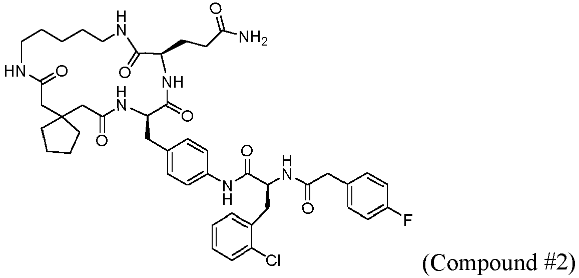 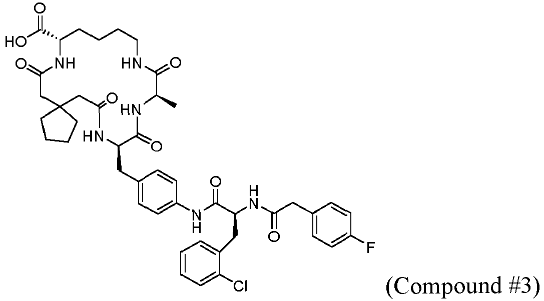 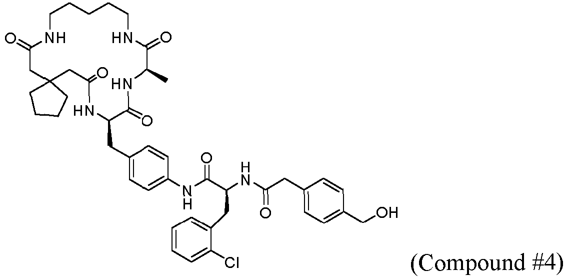 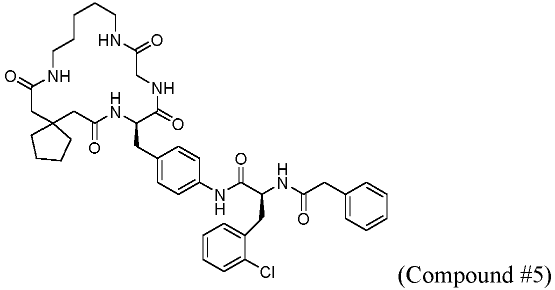 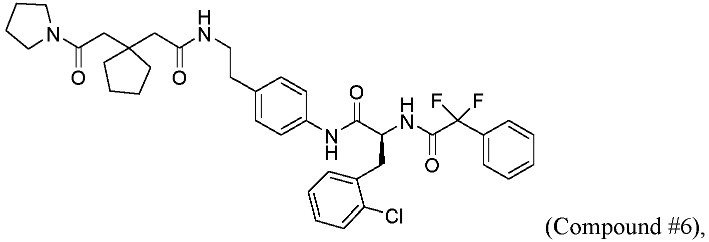 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent. The methods comprise: a) determining the amount of free IL-17 in the sample using the first capture antibody and the first detection agent, as described above; b) determining the amount of total IL-17 in the sample using the second capture antibody and the second detection agent, as described above; and c) subtracting the amount of the free IL-17 determined in step (a) from the amount of total IL-17 in step (b) to obtain the amount of the modulator-bound IL-17 in the sample.

By measuring the amount of modulator-bound IL-17 in a sample, the specificity and target engagement of the SM modulator can be assessed.

As previously stated, the term "small molecule modulator(s)" and/or "SM modulator(s)" and/or "SM modulator of IL-17" refers to any small molecule compound that can bind to IL-17 and has a molecular weight ranging from about 100 g/mol to about 1500 g/mol, or from 400 g/mol to about 1050 g/mol, or from 500 g/mol to about 1000 g/mol, or from about 600 g/mol to about 900 g/mol, or from about 300 g/mol to about 750 g/mol, or from about 500 to about 800 g/mol. Alternatively, the SM modulators may have a molecular weight ranging from a lower limit of about 100 g/mol or 200 g/mol or about 300 g/mol or about 400 g/mol or about 500 g/mol or about 600 g/mol to a upper limit of about 400 g/mol or about 500 g/mol or about 600 or about 700 g/mol or about 800 g/mol or about 900 g/mol or about 1000 g/mol or about 1100 g/mol, or about 1200 g/mol or about 1300 g/mol or about 1400 g/mol or about 1500 g/mol.The SM modulators of IL-17 used herein are selected from compounds #1-6 listed in Table 4 below and pharmaceutically acceptable salts thereof:

**Table 4. Exemplary Small Molecule Modulators of IL-17**

| SM modulator | Structure | Reference | MW (g/mol) |
|---|---|---|---|
| Compound #1 | 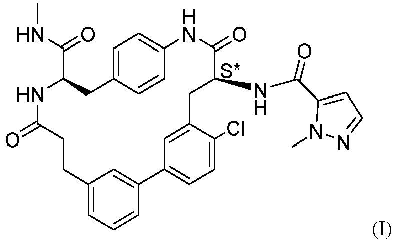 | Compound 1 disclosed as a mixture of diastereomers in "Binding site elucidation and structure guided design of macrocyclic IL-17A antagonists," Scientific Reports, 6, DOI: 10.1038/srep30859 (2016) | 613.12 |
| Compound #2 | 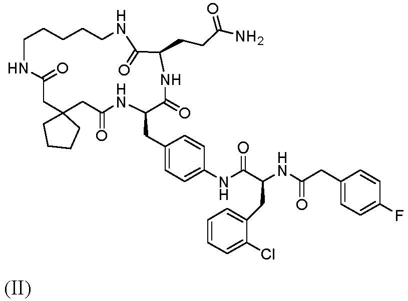 | Compound 479 in WO 2013/116682 | 860.42 |
| Compound #3 | 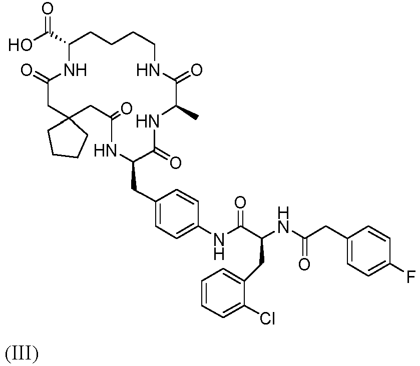 | Compound 529 in WO 2013/116682 | 847.38 |
| Compound #4 | 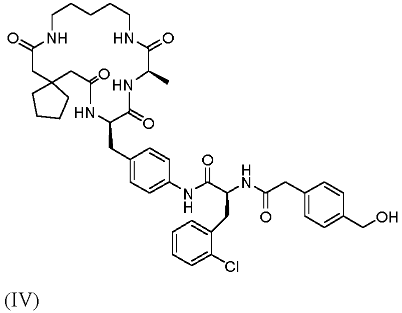 | Compound 487 in WO 2013/116682 | 815.41 |
| Compound #5 | 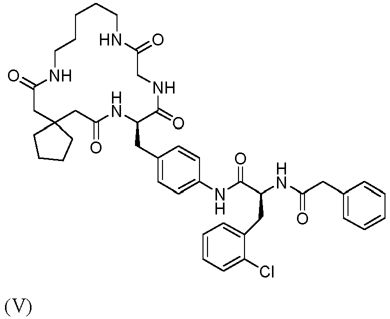 | Compound 159 in WO 2013/116682 | 771.35 |
| Compound #6 | 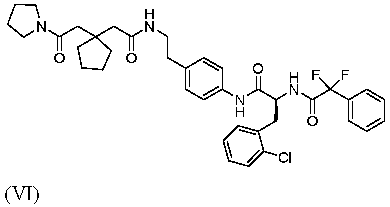 | Compound 286 in WO 2014/066726 | 693.23 |

The following compound: 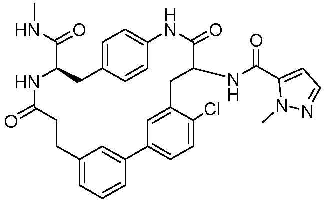 is disclosed in Liu, S. et al. "Binding site elucidation and structure guided design of macrocyclic IL-17A antagonists," Scientific Reports, 6 30859, DOI: 10.1038/srep30859 (2016). This compound was prepared as a mixture of diastereomers, which were separated by supercritical fluid chromatography (SFC) (column: DAICEL CHIRALPAK AD (250 mm * 30 mm, 10 um); mobile phase: [0.1% NH₃H₂O IPA]; B%: 40% - 40%,5.3 min ; 420 min). The more potent diastereomer, assigned herein as Compound #1: 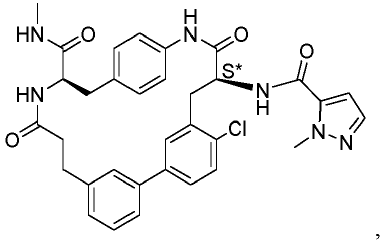 was evaluated using the method disclosed herein.

Compounds #2-5 are disclosed in PCT Patent Application Publication No. WO 2013/116682, titled: "Macrocyclic Compounds for Modulating IL-17,"(see, e.g., Figure 12, compounds 479, 529, 487, and 159, respectively).

Compound #6 is disclosed in PCT Patent Application Publication No. WO 2014/066726, titled: "Compounds for Modulating IL-17," (see, *e.g.,* Figure 6, compound 286).

In certain embodiments, the method disclosed herein achieves a detection sensitivity with Lower Limit of Quantification (LLOQ) at about 0.01 or about 0.1 or about 0.25 or about 0.5 or about 0.75, or 1 pg/ml of the IL-17 in the sample.

In certain embodiments, the IL-17 is a human IL-17.

In certain embodiments, the sample is a biological sample obtained from a human subject under an *ex vivo* or *in vivo* treatment with a SM modulator of human IL-17.

In certain embodiments, the sample may be a biological sample obtained from an animal (e.g., mouse) having human IL-17 injected therein.

In certain embodiments, the biological sample is selected from the group consisting of a sample prepared from cells, tissues, or a serum, plasma or another biological fluid sample.

According to the embodiments of the present application, the method further comprises adjusting the treatment regimen for a subject based on the determined amount of free IL-17 in the biological sample from the subject prior to and after administration of the SM modulator to the subject. For example, the adjustment can be increasing or decreasing the dosage or frequency of the treatment with the SM modulator or switching to a different modulator if the currently used SM modulator is found to bind to IL-17 below a threshold level. The treatment regimen also can be adjusted based on the determined amount of free IL-17 in the biological sample from the subject at two or more time points after administration of the SM modulator to the subject.

In some embodiments, the subject suffers from an IL-17-mediated inflammatory disease. The IL-17-mediated inflammatory disease may be selected from psoriasis, psoriatic arthritis, rheumatoid arthritis, ankylosing spondylitis, hidradenitis suppurativa, bullous pemphigold, atopic dermatitis, vitiligo, multiple sclerosis, asthma, uveitis, chronic obstructive pulmonary disorder, multiple myeloma, and systemic lupus erythematosus. In some embodiments, the IL-17 mediated inflammatory disease is selected from psoriasis, psoriatic arthritis, and ankylosing spondylitis. In some embodiments, the IL-17 mediated inflammatory disease is psoriasis. In some embodiments, the IL-17 mediated inflammatory disease is psoriatic arthritis. In some embodiments, the IL-17 mediated inflammatory disease is ankylosing spondylitis.

In some embodiments, the inventive IL-17 target engagement assay disclosed herein can be used in pre-clinical models for modeling doses in human trials, in human phase 1 trials for decisions on phase 2 dose in patients and for decisions on whether improved target engagement is needed for backup molecules or trial redesign. The correlation of target engagement assay with efficacy in patient phase 2 trials can be used for decision on whether improved target engagement is needed for better efficacy.

The inventive assay can be used to measure free IL-17 in the presence of a small molecule IL-17 modulator(s) independent of any readouts on disease. The assay can be applied to healthy subjects or patients of an IL-17 mediated inflammatory disease. Target engagement readouts and test molecule PK can be used in modeling to determine the optimal dose to achieve efficacy and minimize signals in safety studies.

The present application yet further provides a kit for determining an amount of free IL-17 in a sample comprising the IL-17 and a SM modulator, the kit comprising:
i. a first capture antibody, wherein upon contact with the sample, the first capture antibody forms a first complex comprising the first capture antibody and the IL-17 unbound to the SM modulator, and a second complex comprising the first capture antibody and the IL-17 bound to the SM modulator, respectively; and
ii. a first detection agent (such as first detection antibody), wherein upon contacting with the first complex and the second complex, the first detection agent forms a third complex comprising the first capture antibody, the IL-17 unbound to the SM modulator, and the first detection agent, but not a fourth complex comprising the first capture antibody, the IL-17 bound to the SM modulator, and the first detection agent
wherein the SM modulator is selected from the group consisting of Compounds #1-6 having the following structures and pharmaceutically acceptable salts thereof: 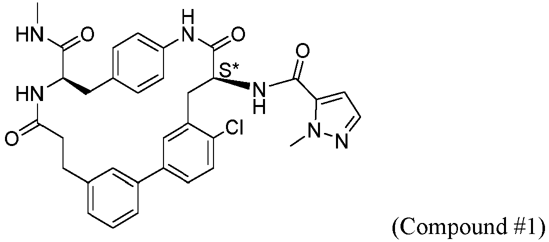 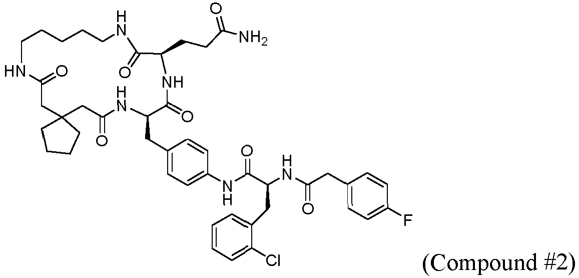 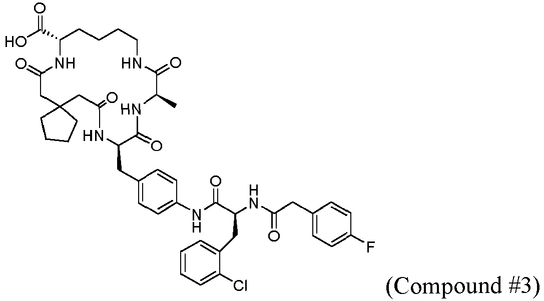 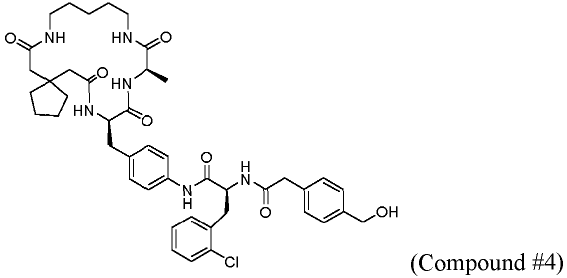 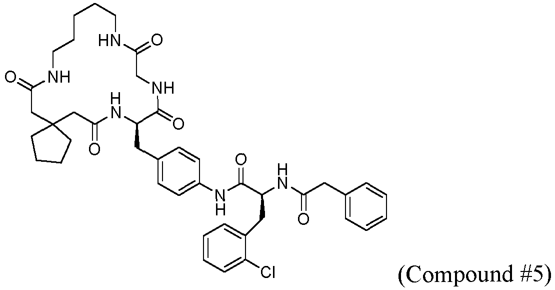 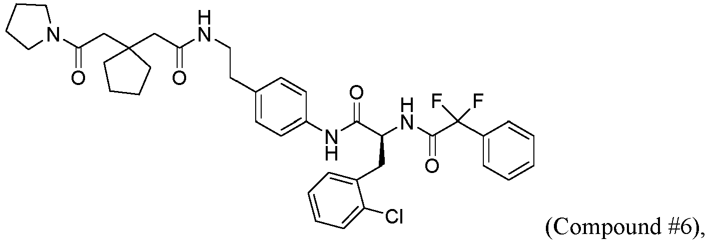
i. the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

In some embodiments, the kit further comprises one or more reagents for detecting the first detection agent.

The present application yet further provides a kit for determining an amount of modulator-bound IL-17 in a sample comprising the IL-17 and a SM modulator, the kit comprising:
i. a first capture antibody, wherein upon contact with the sample, the first capture antibody forms a first complex comprising the first capture antibody and the IL-17 unbound to the SM modulator, and a second complex comprising the first capture antibody and the IL-17 bound to the SM modulator;
ii. a first detection agent (such as a first detection antibody), wherein upon contact with the first complex and the second complex, the first detection agent, forms a third complex comprising the first capture antibody, the IL-17 unbound to the SM modulator, and the first detection agent, but not a fourth complex comprising the first capture antibody, the IL-17 bound to the SM modulator, and the first detection agent; and
iii. a second capture antibody, wherein upon contact with the sample, the second capture antibody forms a fifth complex comprising the second capture antibody and the IL-17 unbound to the SM modulator, and a sixth complex comprising the second capture antibody and the IL-17 bound to the SM modulator;
iv. a second detection agent (such as a second detection antibody), wherein upon contact with the fifth complex and the sixth complex, the second detection agent, forms a seventh complex comprising the second capture antibody, the IL-17 unbound to the SM modulator, and the second detection agent, and an eighth complex comprising the second capture antibody, the IL-17 bound to the SM modulator, and the second detection agent, respectively
wherein the SM modulator is selected from the group consisting of Compounds #1-6 having the following structures and pharmaceutically acceptable salts thereof: 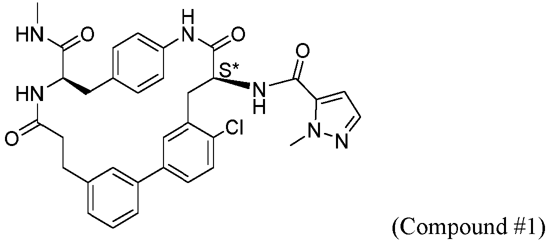 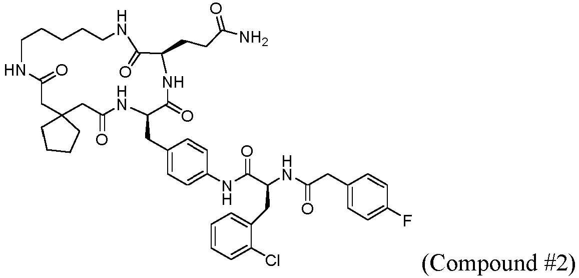 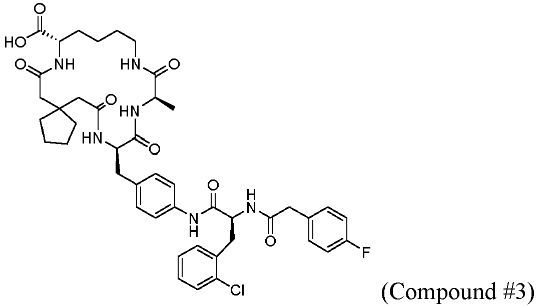 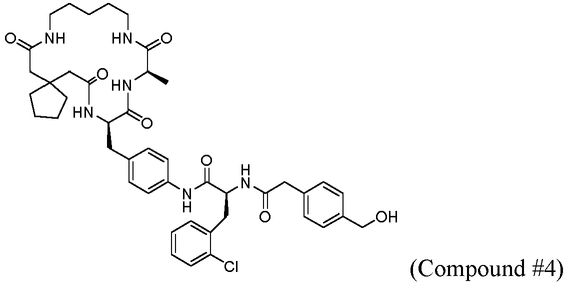 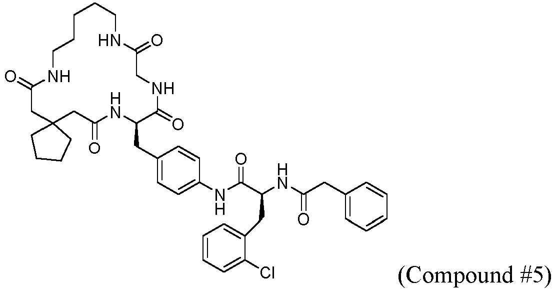 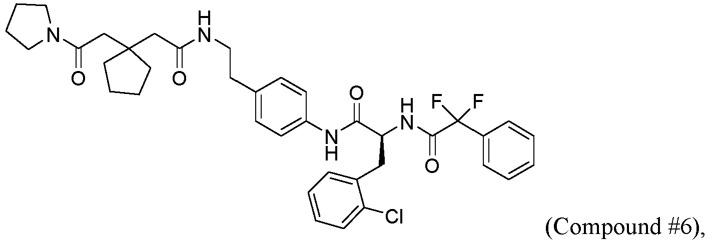 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

In some embodiments, the kit further comprises one or more reagents for detecting the first detection agent and/or second detection agent.

### EXAMPLES

### Example 1

### Materials

Three (3) capture antibody/detection agent pairs (listed in Table 5 below) were tested using Compounds #1-6 (as described above in Table 5) as the SM modulators.

**Table 5**

| **Capture antibody** | | **Detection agent** | |
|---|---|---|---|
| **Company** | **CAT# or ID** | **Company** | **CAT# or ID** |
| R&D Systems, Inc. | Capture antibody of DuoSet ELISA kit DY317 | Janssen Biotech, Inc. | mAb4538 |
| R&D Systems, Inc. | Capture antibody of DuoSet ELISA kit DY317 | R&D Systems, Inc. | Detection antibody of DuoSet ELISA kit DY317 |
| R&D Systems, Inc. | Capture antibody of DuoSet ELISA kit DY317 | CovalAb R&D (Antibodies Online) | antibody ABIN1724556 |

### Methods

Two-fold and 8-point serial dilutions of recombinant IL-17 were prepared in diluent starting from 1 ng/ml, in duplicate. For testing differentiating property on SM modulators, IL-17 calibration samples were prepared in the presence of 10 µM SM modulators or vehicle control containing 0.1% DMSO. IL-17 samples were incubated for 1 hour at 37 °C.

High binding 96-well plates were coated with 100 µL/well of 4 µg/ml capture antibody in PBS overnight at room temperature. Coated plates were washed 6 x 400 µL/well on plate washer (Zoom HT Microplate Washer) and blocked with 300 µL/well blocker (1% BSA in PBS) at room temperature for 2.5 hours followed by 6 x 400 µL/well on plate washer.

Samples were prepared by incubating titrated IL-17 with SM modulators at final concentration of 10 µM. IL-17 levels were measured in ELISA assay by adding 95 µL/well samples to the capture antibody coated plates. Plates were kept at room temperature for 30 minutes and then 5 µL/well of detecting agent were added to a final of 500 ng/mL, kept at room temperature for another 30 minutes, followed by washing 6 x 400 µL/well on plate washer. Development of color for measurement was performed by adding streptavidin conjugated horseradish peroxidase at 100 µL/well and incubated for 20 minutes at room temperature in the dark, followed by 6 x 400 µL/well on plate washer, and then addition of 100 µL/well pf substrate solution, incubated for 20 minutes at room temperature in the dark. Reaction was then stopped by addition of 50 µL/well of stop solution and plates were measured in plate reader at 450-540 nm.

Optical density (OD) readout from plate reader (Molecular Devices Spectra Max 340fPC) were analyzed with Softmax Pro6.3 and GraphPad Prism, and plotted as dose response curve of OD values vs IL-17 concentrations.

### Results

As shown in FIGS 2A-2F, using capture antibody of DuoSet ELISA kit DY317 pairing with mAb4538 (500 ng/ml, as disclosed in U.S. Patent No. 8,519,107) as detection agent, only free IL-17 (that is not SM modulator bound ) could be detected with signals corresponding to the concentration of IL-17, but not SM modulator-bound IL-17.

However, using DuoSet ELISA kit DY317 or using the capture antibody of DuoSet ELISA kit DY317 pairing with antibody ABIN1724556 (CovalAb R&D (Antibodies Online)), total IL-17 (in free and modulator-bound forms) were detected in a dose dependent manner (FIGS 3A-3F and FIGS 4A-4F).

### Example 2

By similar ELISA assay as described in Example 1, various capture antibody/detection agent pairs (with capture antibodies selected from Table 6 and detection agents selected from Table 7) were tested using Compound #1 as the SM modulator. Table 8 below listed the capture antibody/detection agent pairs that were identified by this experiment, which can differentiate free human IL-17 from SM modulator-bound human IL-17. Table 9 below listed the capture antibody/detection agent pairs that cannot differentiate free human IL-17 from SM modulator-bound human IL-17.

**Table 6. Human IL-17 Capture Antibodies**

| **Company** | **CAT# or ID** | **CLONE** |
|---|---|---|
| R&D Systems, Inc. | Capture antibody of DuoSet ELISA kit DY317 | Monoclonal Mouse IgG2B Clone # 41809 (Catalog Number: MAB317) |
| Quanterix Corp. | Capture antibody of IL-17A Advantage Kit 101599 | Ab clone unknown, Simoa bead coated capture antibody |
| Janssen Biotech, Inc. | mAb7024 | N/A |

**Table 7. Screening of antibodies useful as human IL-17 detection agents**

| **Company** | **CAT# or ID** | **CLONE** |
|---|---|---|
| R&D Systems, Inc. | Detection antibody of DuoSet ELISA kit DY317 | Antigen Affinity-purified Polyclonal Goat IgG (Catalog Number: BAF317) |
| Thermo Fisher Scientific, Inc. | Detection antibody of HS ELISA human IL-17A Kit BMS2017HS | Unspecified |
| NOVUS Biologicals LLC | Detection antibody of HS ELISA human IL 17A Kit #NBP1-82423 | Unspecified |
| R&D Systems, Inc. | Detection antibody of human IL-17 HS ELISA Kit HS170 | Unspecified |
| R&D Systems, Inc. (Novus product) | MAB317 | 41809, mouse IgG2b |
| Abiocode | M0002-1 | 7A7, mouse antibody |
| Abnova | H00003605-01 | 3G5, mouse antibody |
| Abnova | ABIN791457 (MAB8821) | 5C94, mouse antibody |
| Antibodies Online (ABO) | ABIN2741968 | 3.36.2.7.3, mouse antibody |
| Biolegend, Inc. | 512702 | BL23, mouse antibody |
| Biorbyt | orb376441 | 3F3-H5-E4 |
| Biorbyt | orb376437 | 1B1-G2-E10 |
| Janssen Biotech, Inc. | mAb8575 | N/A |
| Janssen Biotech, Inc. | mAb3584 | N/A |
| Janssen Biotech, Inc. | mAb3077 | N/A |
| Janssen Biotech, Inc. | mAb732 | N/A |
| Janssen Biotech, Inc. | mAb7024 | N/A |
| Janssen Biotech, Inc. | mAb4538 | N/A |
| Janssen Biotech, Inc. | mAb5548 | N/A |
| Covalab R&D (Antibodies Online) | antibody ABIN1724556 | B-C49, mouse antibody |
| Creative Diagnostics, Inc. | Anti-IL 17A monoclonal antibody DCABH-4543 | 4.52.3.7.2, mouse antibody |
| Creative Diagnostics, Inc. | Anti-IL 17A monoclonal antibody CABT-ZC1183 | 5E28, mouse antibody |
| Creative Diagnostics, Inc. | CABT-L259 | 52910, mouse antibody |
| Creative Diagnostics, Inc. | Anti-IL 17A monoclonal antibody CABT-ZC1184 | 5D28, mouse antibody |
| Creative Diagnostics, Inc. | Anti-IL 17A monoclonal antibody CABT-WN1669 | CM34, mouse antibody |
| Creative Diagnostics, Inc. | Anti-IL 17A monoclonal antibody DCABH-3098 | 8B8, mouse antibody |
| Creative Diagnostics, Inc. | Anti-IL 17A polyclonal antibody CABT-WN1801 | goat polyclonal antibody |
| Invitrogen | IL-17A antibody MA1069B | 3.41.2.6.1, mouse antibody |
| Mabtech AB | Anti-human IL-17A antibody 3520-5N-500 | MT504, mouse antibody |
| Mabtech AB | Anti-human IL-17 antibody 3520-3-250 | MT44.6, mouse antibody |
| My Biosource (MBS) | MBS592444 | 3H11, mouse antibody |
| My Biosource (MBS) | MBS592443 | 3C1, mouse antibody |
| My Biosource (MBS) | MBS592445 | 5A9, mouse antibody |
| My Biosource (MBS) | MBS592447 | 7F10, mouse antibody |
| My Biosource (MBS) | MBS592430 | 4C1, mouse antibody |
| My Biosource (MBS) | MBS592442 | 1E2, mouse antibody |
| My Biosource (MBS) | MBS689608 | no info, mouse antibody |
| My Biosource (MBS) | MBS689609 | no info, mouse antibody |
| My Biosource (MBS) | MBS2542255 | 4679, mouse antibody |
| My Biosource (MBS) | MBS592432 | 8H7, mouse antibody |
| NOVUS Biologicals LLC | IL-17/IL-17A antibody DDX0336B-100 | 408H6.01 |
| NOVUS Biologicals LLC | NBP2-24765B | 4k5F6, mouse antibody |
| OriGene Technologies Inc. | DDX0335B-50 | 414A11.03 |
| OriGene Technologies Inc. | DDX0334B-50 | 412G6.06 |
| OriGene Technologies Inc. | AM31338BT-N | B-B51, mouse antibody |
| ProSci | 36-072 | 17-142F, mouse antibody |
| Ray Biotech | 130-10382-200 | 3A9-G5-A6, mouse antibody |
| Rockland Immunochemicals Inc. | IL-17A biotin conjugated antibody 209-306-B32 | 4H1524.1, mouse antibody |
| Sino Biological Inc. | ABIN5507411 | 6, mouse antibody |
| Wuhan Fine Biotech | ABIN5698147 | mouse antibody |
| R&D Systems, Inc. | Recombinant Human IL-17RA/IL-17R Fc Chimera Protein, CF, 177-IR-100 | rhIL 17RA/IL 17-R Fc |
| Sino Biological Inc. | 10895-H08H-B | IL-17RA (monomer) |

**Table 8. Capture Antibody/Detection Agent pairs that detect only free human IL-17 in the presence of SM modulators**

| **Capture Antibody** | | | **Detection Agent** | | |
|---|---|---|---|---|---|
| **Company** | **CAT# or ID** | **Clone** | **Company** | **CAT# or ID** | **Clone** |
| R&D Systems, Inc. | Capture antibody of DuoSet ELISA kit DY317 | Monoclonal Mouse IgG2B Clone # 41809 (Catalog Number: MAB317) | Thermo Fisher Scientific, Inc. | Detection antibody of HS ELISA human IL-17A Kit BMS2017HS | eBioscience HS ELISA human IL17A Kit detection antibody |
| R&D Systems, Inc. | Capture antibody of DuoSet ELISA kit DY317 | Monoclonal Mouse IgG2B Clone # 41809 (Catalog Number: MAB317) | NOVUS Biologicals LLC | Detection antibody of HS ELISA human IL17A Kit #NBP1-82423 | NOVUS HS ELISA human IL 17A Kit detection antibody |
| Janssen Biotech, Inc. | mAb7024* | N/A | NOVUS Biologicals LLC | Detection antibody of HS ELISA human IL17A Kit #NBP1-82423 | NOVUS HS ELISA human IL 17A Kit detection antibody |
| R&D Systems, Inc. | Capture antibody of DuoSet ELISA kit DY317 | Monoclonal Mouse IgG2B Clone # 41809 (Catalog Number: MAB317) | BioLegend, Inc. | Anti-human IL-17A antibody 512702 | BL23, mouse antibody |
| R&D Systems, Inc. | Capture antibody of DuoSet ELISA kit DY317 | Monoclonal Mouse IgG2B Clone # 41809 (Catalog Number: MAB317) | Janssen Biotech, Inc. | mAb3584* | N/A |
| Quanterix Corp. | Capture antibody of Il-17A Advantage Kit 101599 | Quanterix Corp. IL 17A kit bead coated capture antibody | Janssen Biotech, Inc. | mAb3584* | N/A |
| R&D Systems, Inc. | Capture antibody of DuoSet ELISA kit DY317 | Monoclonal Mouse IgG2B Clone # 41809 (Catalog Number: MAB317) | Janssen Biotech, Inc. | mAb3077* | N/A |
| Quanterix Corp. | Capture antibody of Il-17A Advantage Kit 101599 | Quanterix Corp. IL 17A kit bead coated capture antibody | Janssen Biotech, Inc. | mAb3077* | N/A |
| R&D Systems, Inc. | Capture antibody of DuoSet ELISA kit DY317 | Monoclonal Mouse IgG2B Clone # 41809 (Catalog Number: MAB317) | Janssen Biotech, Inc. | mAb732* | N/A |
| Quanterix Corp. | Capture antibody of Il-17A Advantage Kit 101599 | Quanterix Corp. IL 17A kit bead coated capture antibody | Janssen Biotech, Inc. | mAb732* | N/A |
| R&D Systems, Inc. | Capture antibody of DuoSet ELISA kit DY317 | Monoclonal Mouse IgG2B Clone # 41809 (Catalog Number: MAB317) | Janssen Biotech, Inc. | mAb7024* | N/A |
| R&D Systems, Inc. | Capture antibody of DuoSet ELISA kit DY317 | Monoclonal Mouse IgG2B Clone # 41809 (Catalog Number: MAB317) | Janssen Biotech, Inc. | mAb4538* | N/A |
| R&D Systems, Inc. | Capture antibody of DuoSet ELISA kit DY317 | Monoclonal Mouse IgG2B Clone # 41809 (Catalog Number: MAB317) | Janssen Biotech, Inc. | mAb5548* | N/A |
| R&D Systems, Inc. | Capture antibody of DuoSet ELISA kit DY317 | Monoclonal Mouse IgG2B Clone # 41809 (Catalog Number: MAB317) | Creative Diagnostics, Inc. | Anti-IL 17A monoclonal antibody DCABH-4543 | 4.52.3.7.2, mouse antibody |
| R&D Systems, Inc. | Capture antibody of DuoSet ELISA kit DY317 | Monoclonal Mouse IgG2B Clone # 41809 (Catalog Number: MAB317) | Creative Diagnostics, Inc. | Anti-IL 17A monoclonal antibody CABT-ZC1183 | 5E28, mouse antibody |
| R&D Systems, Inc. | Capture antibody of DuoSet ELISA kit DY317 | Monoclonal Mouse IgG2B Clone # 41809 (Catalog Number: MAB317) | Creative Diagnostics, Inc. | Anti-IL 17A monoclonal antibody CABT-WN1669 | CM34, mouse antibody |
| R&D Systems, Inc. | Capture antibody of DuoSet ELISA kit DY317 | Monoclonal Mouse IgG2B Clone # 41809 (Catalog Number: MAB317) | Invitrogen | IL-17A antibody MA 1069B | 3.41.2.6.1, mouse antibody |
| Quanterix Corp. | Capture antibody of Il-17A Advantage Kit 101599 | Quanterix Corp. IL17A kit bead coated capture antibody | Invitrogen | IL-17A antibody MA 1069B | 3.41.2.6.1, mouse antibody |
| Janssen Biotech, Inc. | mAb7024* | N/A | Invitrogen | IL-17A antibody MA 1069B | 3.41.2.6.1, mouse antibody |
| R&D Systems, Inc. | Capture antibody of DuoSet ELISA kit DY317 | Monoclonal Mouse IgG2B Clone # 41809 (Catalog Number: MAB317) | Mabtech AB | Anti-human IL-17A antibody 3520-5N-500 | MT504, mouse antibody |
| Janssen Biotech, Inc. | mAb7024* | N/A | Mabtech AB | Anti-human IL-17A antibody 3520-5N-500 | MT504, mouse antibody |
| R&D Systems, Inc. | Capture antibody of DuoSet ELISA kit DY317 | Monoclonal Mouse IgG2B Clone # 41809 (Catalog Number: MAB317) | Sino Biological Inc. | Antibody ABIN5507411 | 6, mouse antibody |
| R&D Systems, Inc. | Capture antibody of DuoSet ELISA kit DY317 | Monoclonal Mouse IgG2B Clone # 41809 (Catalog Number: MAB317) | R&D Systems, Inc. | Recombinant Human IL-17RA/IL-17R Fc Chimera Protein, CF, 177-IR-100 | rhIL 17RA/IL 17-R Fc |
| R&D Systems, Inc. | Capture antibody of DuoSet ELISA kit DY317 | Monoclonal Mouse IgG2B Clone # 41809 (Catalog Number: MAB317) | Sino Biological Inc. | IL-17RA protein 10895-H08H-B | IL-17RA (monomer) |

| | | | | | |
|---|---|---|---|---|---|
| * as disclosed in U.S. Patent No. 8,519,107 | | | | | |

**Table 9. Capture Antibody/Detection Agent pairs that detect both free and modulator-bound IL-17 in the presence of SM modulator**

| **Capture Antibody** | | | **Detection Agent** | | |
|---|---|---|---|---|---|
| **Company** | **CAT# or ID** | **Clone** | **Company** | **CAT# or ID** | **Clone** |
| R&D Systems, Inc. | Capture antibody of DuoSet ELISA kit DY317 | Monoclonal Mouse IgG2B Clone # 41809 (Catalog Number: MAB317) | R&D Systems, Inc. | Detection antibody of DuoSet ELISA kit DY317 | Antigen Affinity-purified Polyclonal Goat IgG (Catalog Number: BAF317) |
| R&D Systems, Inc. | Capture antibody of DuoSet ELISA kit DY317 | Monoclonal Mouse IgG2B Clone # 41809 (Catalog Number: MAB317) | R&D Systems, Inc. | Detection antibody of human IL-17 HS ELISA Kit HS170 | R&D Quantikine HS ELISA human IL17A kit detection antibody |
| R&D Systems, Inc. | Capture antibody of DuoSet ELISA kit DY317 | Monoclonal Mouse IgG2B Clone # 41809 (Catalog Number: MAB317) | Janssen Biotech, Inc. | mAb8575 | N/A |
| R&D Systems, Inc. | Capture antibody of DuoSet ELISA kit DY317 | Monoclonal Mouse IgG2B Clone # 41809 (Catalog Number: MAB317) | CovalAb R&D (Antibodies Online) | antibody ABIN1724556 | B-C49, mouse antibody |
| R&D Systems, Inc. | Capture antibody of DuoSet ELISA kit DY317 | Monoclonal Mouse IgG2B Clone # 41809 (Catalog Number: MAB317) | Creative Diagnostics, Inc. | CABT-ZC1184 | 5D28, mouse antibody |
| R&D Systems, Inc. | Capture antibody of DuoSet ELISA kit DY317 | Monoclonal Mouse IgG2B Clone # 41809 (Catalog Number: MAB317) | Mabtech AB | Anti-human IL-17 antibody 3520-3-250 | MT44.6, mouse antibody |
| R&D Systems, Inc. | Capture antibody of DuoSet ELISA kit DY317 | Monoclonal Mouse IgG2B Clone # 41809 (Catalog Number: MAB317) | My Biosource (MBS) | MBS592444 | 3H11, mouse antibody |
| R&D Systems, Inc. | Capture antibody of DuoSet ELISA kit DY317 | Monoclonal Mouse IgG2B Clone # 41809 (Catalog Number: MAB317) | My Biosource (MBS) | MBS592430 | 4C1, mouse antibody |
| R&D Systems, Inc. | Capture antibody of DuoSet ELISA kit DY317 | Monoclonal Mouse IgG2B Clone # 41809 (Catalog Number: MAB317) | My Biosource (MBS) | MBS689609 | no info, mouse antibody |
| R&D Systems, Inc. | Capture antibody of DuoSet ELISA kit DY317 | Monoclonal Mouse IgG2B Clone # 41809 (Catalog Number: MAB317) | My Biosource (MBS) | MBS592432 | 8H7, mouse antibody |
| R&D Systems, Inc. | Capture antibody of DuoSet ELISA kit DY317 | Monoclonal Mouse IgG2B Clone # 41809 (Catalog Number: MAB317) | NOVUS Biologicals LLC | IL-17/IL-17A antibody DDX0336B-100 | 408H6.01 |
| R&D Systems, Inc. | Capture antibody of DuoSet ELISA kit DY317 | Monoclonal Mouse IgG2B Clone # 41809 (Catalog Number: MAB317) | OriGene Technologies Inc. | DDX0335B-50 | 414A11.03 |
| R&D Systems, Inc. | Capture antibody of DuoSet ELISA kit DY317 | Monoclonal Mouse IgG2B Clone # 41809 (Catalog Number: MAB317) | OriGene Technologies Inc. | DDX0334B-50 | 412G6.06 |
| R&D Systems, Inc. | Capture antibody of DuoSet ELISA kit DY317 | Monoclonal Mouse IgG2B Clone # 41809 (Catalog Number: MAB317) | Rockland Immunochemicals Inc. | IL-17A biotin conjugated antibody 209-306-B32 | 4H1524.1, mouse antibody |

### Example 3

In this example, samples containing 1000 pg/ml of IL-17 with titrated SM modulators were assayed using the capture antibody of DuoSet ELISA kit DY317 (from R&D Systems, Inc.) pairing with mAb4538 (from Janssen Biotech, Inc.) as the detection agent. As shown in FIGS. 5A-5B, decreasing levels of free IL-17 were detected with the increasing concentration of compounds, demonstrating a dose-dependent target engagement of IL-17 SM modulators.

## Claims

1. A method of determining an amount of free IL-17 in a sample comprising IL-17 and a small molecule (SM) modulator of IL-17, the method comprising:
i. contacting the sample with a first capture antibody to form a mixture comprising a first complex comprising the first capture antibody and the IL-17 unbound to the SM modulator, and a second complex comprising the first capture antibody and the IL-17 bound to the SM modulator;
ii. contacting the mixture from step i) with a first detection agent to thereby form a third complex comprising the first capture antibody, the IL-17 unbound to the SM modulator, and the first detection agent, but not a fourth complex comprising the first capture antibody, the IL-17 bound to the SM modulator, and the first detection agent; and
iii. determining the amount of the free IL-17 by measuring the amount of the first detection agent in the third complex,
wherein the SM modulator is selected from the group consisting of Compounds #1-6 having the following structures and pharmaceutically acceptable salts thereof: 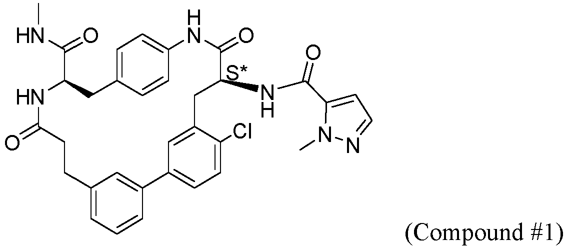 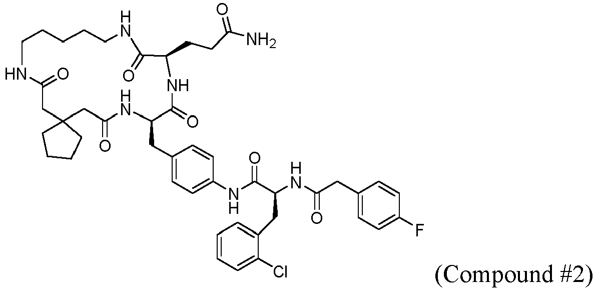 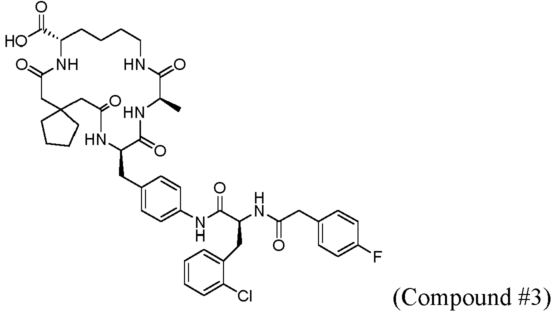 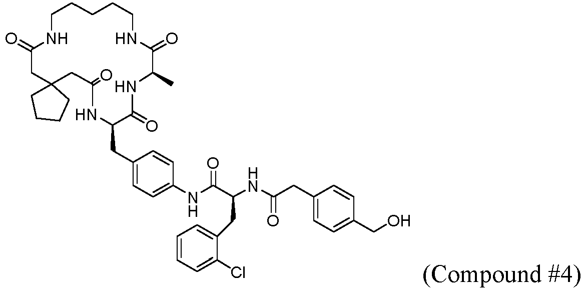 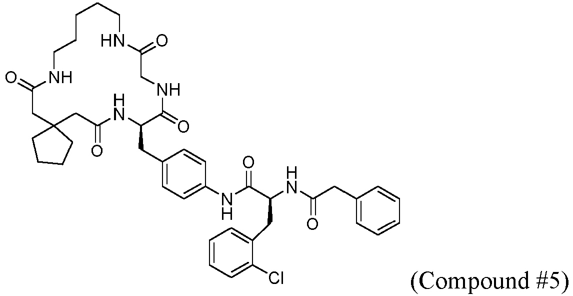 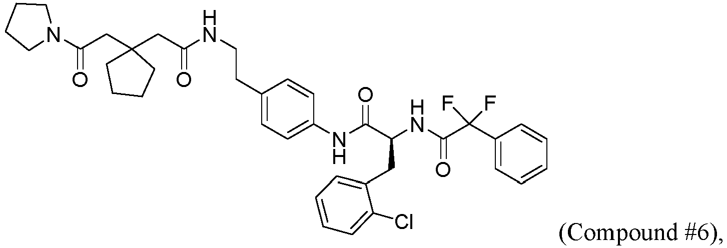 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

2. The method of claim 1, wherein no wash step is performed before contacting the mixture from step i) with the first detection agent.

3. A method of determining the target engagement of a SM modulator of IL-17 in a subject, comprising a) determining the amount of free IL-17 in a sample from the subject before administration of the SM modulator using the method of claim 1; b) determining the amount of free IL-17 in a sample from the subject after administration of the SM modulator using the method of claim 1; and c) comparing the amounts of free IL-17 determined from steps a) and b), wherein the SM modulators are as defined in claim 1.

4. A method of determining the target engagement of a SM modulator of IL-17 in a subject, comprising a) determining the amount of free IL-17 in a sample from the subject at two or more time points after the administration of the SM modulator using the method of claim 1; and b) comparing the amounts of free IL-17 at the two or more time points determined from step a), wherein the SM modulators are as defined in claim 1.

5. A method of determining an amount of modulator-bound IL-17 in a sample comprising IL-17 and a SM modulator of IL-17, the method comprising:
A. determining the amount of free IL-17 using the method of claim 1;
B. contacting a second portion of the sample with a second capture antibody to form a second mixture comprising a fifth complex comprising the second capture antibody and the IL-17 unbound to the SM modulator, and a sixth complex comprising the second capture antibody and the IL-17 bound to the SM modulator;
C. contacting the second mixture with a second detection agent to thereby form a seventh complex comprising the second capture antibody, the IL-17 unbound to the SM modulator, and the second detection agent and an eighth complex comprising the second capture antibody, the IL-17 bound to the SM modulator, and the second detection agent;
D. determining a total amount of the IL-17 by measuring the total amount of the second detection agent in the seventh complex and the eighth complex; and
E. determining the amount of the modulator-bound IL-17 in the sample by subtracting the amount of the free IL-17 determined in step (A) from the total amount of the IL-17 in step (D), wherein the SM modulators are as defined in claim 1.

6. A method of determining the target engagement of a SM modulator of IL-17 in a subject, comprising determining the amount of modulator-bound IL-17 in a sample from the subject after administration of the SM modulator using the method of claim 5, wherein the SM modulators are as defined in claim 1.

7. The method of any one of claims 1-6, wherein the SM modulator is selected from the group consisting of Compounds #2-6 having the following structures and pharmaceutically acceptable salts thereof:

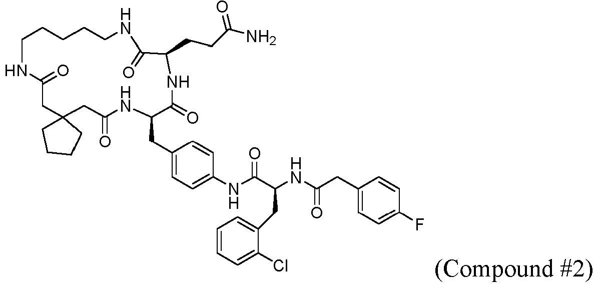

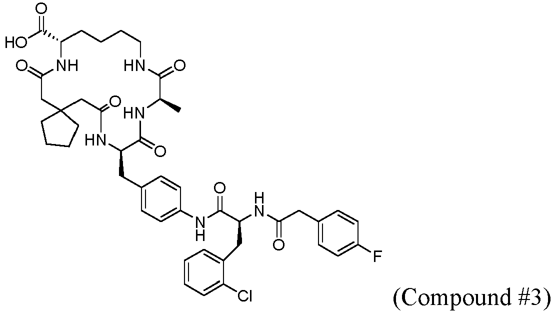

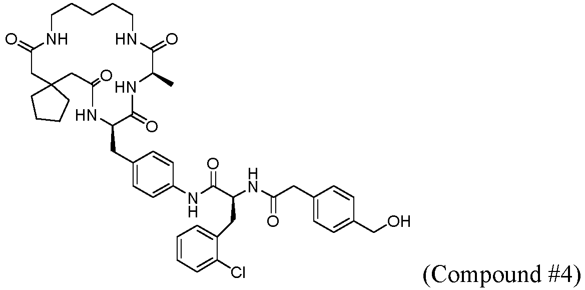

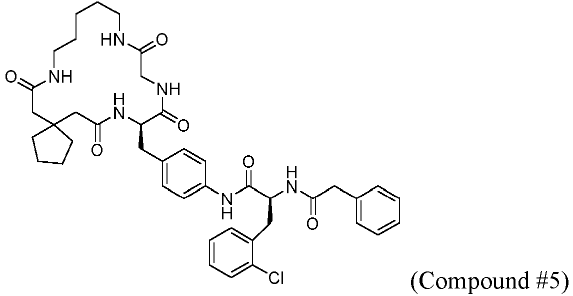

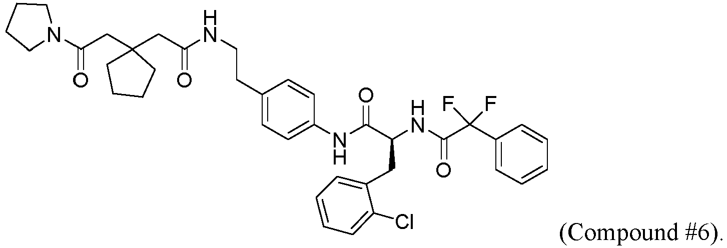

8. The method of any one of claims 1-7, wherein the first and/or the second detection agent is labeled with a detectable label, more preferably, the label is an enzyme or biotin.

9. The method of any one of claims 5-8, wherein the second capture antibody and the second detection agent are selected from the pairs in the following table:
| **Second capture antibody** | | **Second detection agent** | |
|---|---|---|---|
| **Company** | **CAT#** | **Company** | **CAT#** |
| R&D Systems, Inc. | Capture antibody of DuoSet ELISA kit DY317 | R&D Systems, Inc. | Detection antibody of DuoSet ELISA kit DY317 |
| R&D Systems, Inc. | Capture antibody of DuoSet ELISA kit DY317 | R&D Systems, Inc. | Detection antibody of human IL-17 HS ELISA Kit HS170 |
| R&D Systems, Inc. | Capture antibody of DuoSet ELISA kit DY317 | CovalAb R&D (Antibodies Online) | antibody ABIN1724556 |
| R&D Systems, Inc. | Capture antibody of DuoSet ELISA kit DY317 | Creative Diagnostics | Anti-IL 17A monoclonal antibody CABT-ZC1184 |
| R&D Systems, Inc. | Capture antibody of DuoSet ELISA kit DY317 | Mabtech AB | Anti-human IL-17 antibody 3520-3-250 |
| R&D Systems, Inc. | Capture antibody of DuoSet ELISA kit DY317 | MyBioSource (MBS) | Anti-IL-17A antibody MBS592444 |
| R&D Systems, Inc. | Capture antibody of DuoSet ELISA kit DY317 | MyBioSource (MBS) | Anti-IL-17A antibody MBS592430 |
| R&D Systems, Inc. | Capture antibody of DuoSet ELISA kit DY317 | MyBioSource (MBS) | Anti-IL-17A antibody MBS689609 |
| R&D Systems, Inc. | Capture antibody of DuoSet ELISA kit DY317 | MyBioSource (MBS) | Anti-IL-17A antibody MBS592432 |
| R&D Systems, Inc. | Capture antibody of DuoSet ELISA kit DY317 | NOVUS Biologicals LLC | IL-17/IL-17A antibody DDX0336B-100 |
| R&D Systems, Inc. | Capture antibody of DuoSet ELISA kit DY317 | Antibodies Online | ABIN786722 |
| R&D Systems, Inc. | Capture antibody of DuoSet ELISA kit DY317 | OriGene Technologies Inc. | DDX0334B-50 |
| R&D Systems, Inc. | Capture antibody of DuoSet ELISA kit DY317 | OriGene Technologies Inc. | DDX0335B-50 |
| R&D Systems, Inc. | Capture antibody of DuoSet ELISA kit DY317 | Rockland Immunochemicals Inc. | IL-17A biotin conjugated antibody 209-306-B32 |
, optionally wherein the second capture antibody and the second detection agent are capture antibody and detection antibody of DuoSet ELISA kit DY317 (from R&D Systems, Inc.), respectively.

10. The method of any one of claims 1-9, wherein:
a. the sample is a biological sample obtained from a subject under an *ex vivo* or *in vivo* treatment with the SM modulator, optionally wherein:
i. the subject is a mammal, preferably human; and/or
ii. the biological sample is selected from the group consisting of a sample prepared from cells, tissues, or a serum, plasma or another biological fluid sample; and/or
b. the method has a detection sensitivity with Lower Limit of Quantification (LLOQ) at about 0.01 or about 0.1 or about 0.25, or about 0.5, or about 0.75, or about 1 pg/ml of the IL-17 in the sample.

11. A kit for determining an amount of free IL-17 in a sample comprising IL-17 and a SM modulator of IL-17, the kit comprising:
i. a first capture antibody, wherein upon contact with the sample, the first capture antibody forms a first complex comprising the first capture antibody and the IL-17 unbound to the SM modulator, and a second complex comprising the first capture antibody and the IL-17 bound to the SM modulator; and
ii. a first detection agent, wherein upon contact with the first complex and the second complex, the first detection agent, forms a third complex comprising the first capture antibody, the IL-17 unbound to the SM modulator, and the first detection agent, but not a fourth complex comprising the first capture antibody, the IL-17 bound to the SM modulator, and the first detection agent,
wherein the SM modulator is selected from the group consisting of Compounds #1-6 having the following structures and pharmaceutically acceptable salts thereof: 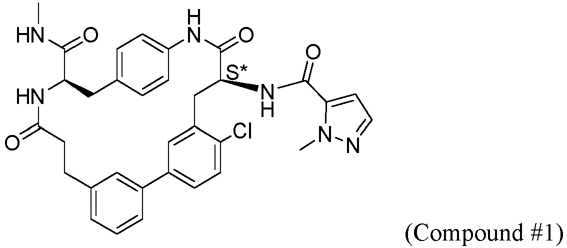 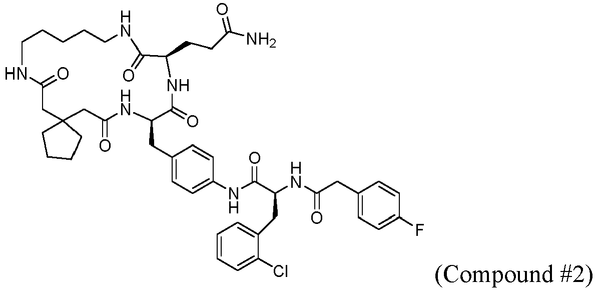 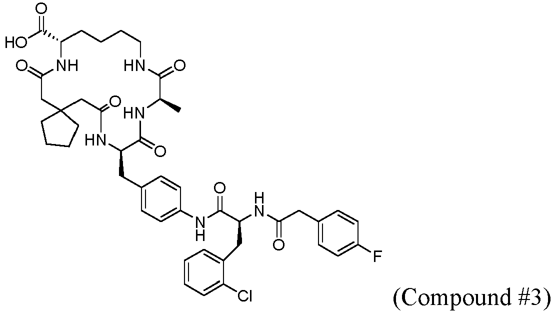 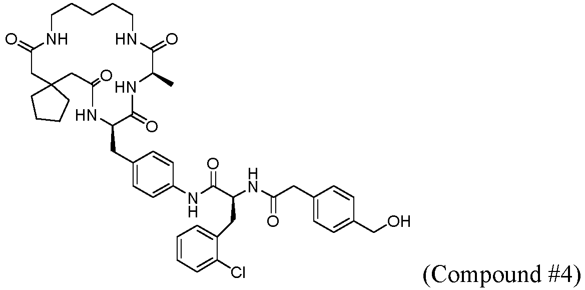 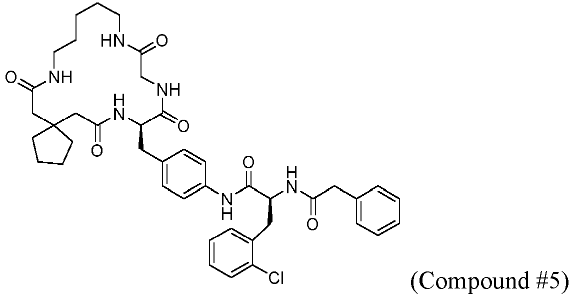 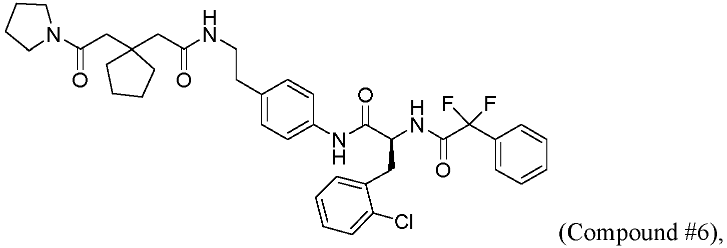 the first capture antibody is the capture antibody of DuoSet ELISA kit DY317 (R&D Systems, Inc.) and the first detection agent is mAb4538 (Janssen Biotech, Inc.) and the first detection agent is not capable of forming a complex comprising the first capture antibody, the IL-17 bound to any of compounds #1 to #6 and the first detection agent.

12. The kit according to claim 11, further comprising:
B. a second capture antibody, wherein upon contact with the sample, the second capture antibody forms a fifth complex comprising the second capture antibody and the IL-17 unbound to the SM modulator, and a sixth complex comprising the second capture antibody and the IL-17 bound to the SM modulator; and
C. a second detection agent, wherein upon contact with the fifth complex and the sixth complex, the second detection agent, forms a seventh complex comprising the second capture antibody, the IL-17 unbound to the SM modulator, and the second detection agent, and an eighth complex comprising the second capture antibody, the IL-17 bound to the SM modulator, and the second detection agent, respectively.

13. The kit of claim 12, wherein the first detection agent and/or the second detection agent, is labeled with a detectable label, more preferably, the label is an enzyme or biotin.

14. The kit of any one of claims 12-13, wherein the second capture antibody and the second detection agent are selected from the pairs in the following table:
| **Second capture antibody** | | **Second detection agent** | |
|---|---|---|---|
| **Company** | **CAT#** | **Company** | **CAT#** |
| R&D Systems, Inc. | Capture antibody of DuoSet ELISA kit DY317 | R&D Systems, Inc. | Detection antibody of DuoSet ELISA kit DY317 |
| R&D Systems, Inc. | Capture antibody of DuoSet ELISA kit DY317 | R&D Systems, Inc. | Detection antibody of human IL-17 HS ELISA Kit HS170 |
| R&D Systems, Inc. | Capture antibody of DuoSet ELISA kit DY317 | CovalAb R&D (Antibodies Online) | antibody ABIN1724556 |
| R&D Systems, Inc. | Capture antibody of DuoSet ELISA kit DY317 | Creative Diagnostics | Anti-IL 17A monoclonal antibody CABT-ZC1184 |
| | | | |
|---|---|---|---|
| R&D Systems, Inc. | Capture antibody of DuoSet ELISA kit DY317 | Mabtech AB | Anti-human IL-17 antibody 3520-3-250 |
| R&D Systems, Inc. | Capture antibody of DuoSet ELISA kit DY317 | MyBioSource (MBS) | Anti-IL-17A antibody MBS592444 |
| R&D Systems, Inc. | Capture antibody of DuoSet ELISA kit DY317 | MyBioSource (MBS) | Anti-IL-17A antibody MBS592430 |
| R&D Systems, Inc. | Capture antibody of DuoSet ELISA kit DY317 | MyBioSource (MBS) | Anti-IL-17A antibody MBS689609 |
| R&D Systems, Inc. | Capture antibody of DuoSet ELISA kit DY317 | MyBioSource (MBS) | Anti-IL-17A antibody MBS592432 |
| R&D Systems, Inc. | Capture antibody of DuoSet ELISA kit DY317 | NOVUS Biologicals LLC | IL-17/IL-17A antibody DDX0336B-100 |
| R&D Systems, Inc. | Capture antibody of DuoSet ELISA kit DY317 | Antibodies Online | ABIN786722 |
| R&D Systems, Inc. | Capture antibody of DuoSet ELISA kit DY317 | OriGene Technologies Inc. | DDX0334B-50 |
| R&D Systems, Inc. | Capture antibody of DuoSet ELISA kit DY317 | OriGene Technologies Inc. | DDX0335B-50 |
| R&D Systems, Inc. | Capture antibody of DuoSet ELISA kit DY317 | Rockland Immunochemicals Inc. | IL-17A biotin conjugated antibody 209-306-B32 |
, optionally wherein the second capture antibody and the second detection agent are capture antibody and detection antibody of DuoSet ELISA kit DY317 (from R&D Systems, Inc.), respectively.

## Patentansprüche

1. Verfahren zum Bestimmen einer Menge an freiem IL-17 in einer Probe, umfassend IL-17 und einen Modulator kleiner Moleküle (SM-Modulator) von IL-17, das Verfahren umfassend:
i. Inkontaktbringen der Probe mit einem ersten Fängerantikörper, um eine Mischung auszubilden, umfassend einen ersten Komplex, umfassend den ersten Fängerantikörper und das IL-17, das an den SM-Modulator ungebunden ist, und einen zweiten Komplex, umfassend den ersten Fängerantikörper und das IL-17, das an den SM-Modulator gebunden ist;
ii. Inkontaktbringen der Mischung aus Schritt i) mit einem ersten Nachweismittel, wobei dadurch ein dritter Komplex ausgebildet wird, umfassend den ersten Fängerantikörper, das IL-17, das an den SM-Modulator ungebunden ist, und das erste Nachweismittel, jedoch keinen vierten Komplex, umfassend den ersten Fängerantikörper, das IL-17, das an den SM-Modulator gebunden ist und das erste Nachweismittel; und
iii. Bestimmen der Menge des freien IL-17 durch Messen der Menge des ersten Nachweismittels in dem dritten Komplex,
wobei der SM-Modulator aus der Gruppe ausgewählt ist, bestehend aus Verbindungen #1-6, die die folgenden Strukturen aufweisen, und pharmazeutisch verträglichen Salzen davon: 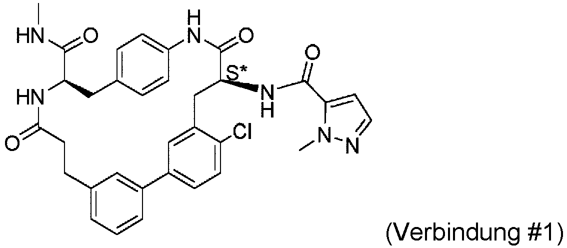 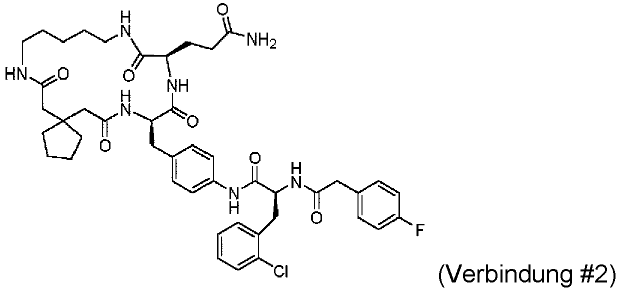 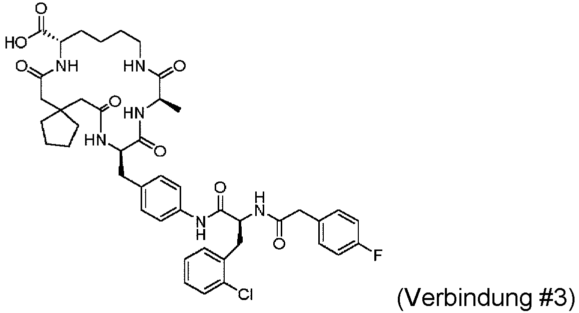 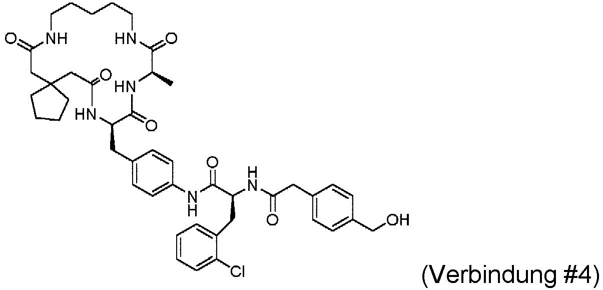 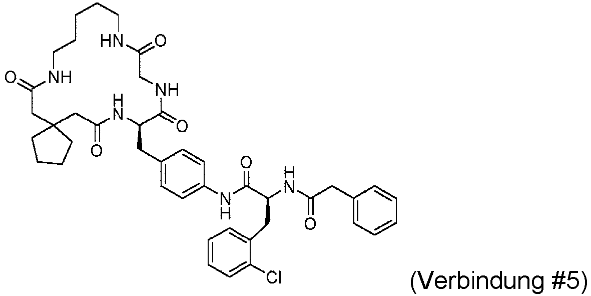 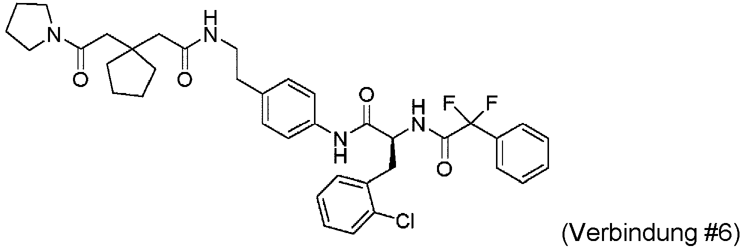
der erste Fängerantikörper der Fängerantikörper eines DuoSet ELISA-Kits DY317 (R&D Systems, Inc.) ist und das erste Nachweismittel mAb4538 (Janssen Biotech, Inc.) ist und das erste Nachweismittel nicht in der Lage ist, einen Komplex auszubilden, umfassend ersten Fängerantikörper, das IL-17, das an eine beliebige der Verbindungen #1 bis #6 gebunden ist und das erste Nachweismittel.

2. Verfahren nach Anspruch 1, wobei vor dem Inkontaktbringen der Mischung aus Schritt i) mit dem ersten Nachweismittel kein Waschschritt durchgeführt wird.

3. Verfahren zum Bestimmen der Zieleinbindung eines SM-Modulators von IL-17 in einem Subjekt, umfassend a) Bestimmen der Menge an freiem IL-17 in einer Probe von dem Subjekt vor einer Verabreichung des SM-Modulators unter Verwendung des Verfahrens nach Anspruch 1; b) Bestimmen der Menge an freiem IL-17 in einer Probe von dem Subjekt nach der Verabreichung des SM-Modulators unter Verwendung des Verfahrens nach Anspruch 1; und c) Vergleichen der Mengen an freiem IL-17, die aus Schritten a) und b) bestimmt werden, wobei die SM-Modulatoren wie in Anspruch 1 definiert sind.

4. Verfahren zum Bestimmen der Zieleinbindung eines SM-Modulators von IL-17 in einem Subjekt, umfassend a) Bestimmen der Menge an freiem IL-17 in einer Probe von dem Subjekt zu zwei oder mehr Zeitpunkten nach der Verabreichung des SM-Modulators unter Verwendung des Verfahrens nach Anspruch 1; und b) Vergleichen der Mengen an freiem IL-17 zu den zwei oder mehr Zeitpunkten, die aus Schritt a) bestimmt werden, wobei die SM-Modulatoren wie in Anspruch 1 definiert sind.

5. Verfahren zum Bestimmen einer Menge an modulatorgebundenem IL-17 in einer Probe, umfassend IL-17 und einen SM-Modulator von IL-17, das Verfahren umfassend:
A. Bestimmen der Menge an freiem IL-17 unter Verwendung des Verfahrens nach Anspruch 1;
B. Inkontaktbringen eines zweiten Abschnitts der Probe mit einem zweiten Fängerantikörper, um eine zweite Mischung auszubilden, umfassend einen fünften Komplex, umfassend den zweiten Fängerantikörper und das IL-17, das an den SM-Modulator ungebunden ist, und einen sechsten Komplex, umfassend den zweiten Fängerantikörper und das IL-17, das an den SM-Modulator gebunden ist;
C. Inkontaktbringen der zweiten Mischung mit einem zweiten Nachweismittel, wobei dadurch ein siebter Komplex ausgebildet wird, umfassend den zweiten Fängerantikörper, das IL-17, das an den SM-Modulator ungebunden ist, und das zweite Nachweismittel, und einen achten Komplex, umfassend den zweiten Fängerantikörper, das IL-17, das an den SM-Modulator gebunden ist, und das zweite Nachweismittel;
D. Bestimmen einer Gesamtmenge an IL-17 durch Messen der Gesamtmenge des zweiten Nachweismittels in dem siebten Komplex und dem achten Komplex; und
E. Bestimmen der Menge des modulatorgebundenen IL-17 in der Probe durch Subtrahieren der Menge des freien IL-17, die in Schritt (A) bestimmt wird, von der Gesamtmenge des IL-17 in Schritt (D), wobei die SM-Modulatoren wie in Anspruch 1 definiert sind.

6. Verfahren zum Bestimmen der Zieleinbindung eines SM-Modulators von IL-17 in einem Subjekt, umfassend das Bestimmen der Menge an modulatorgebundenem IL-17 in einer Probe von dem Subjekt nach der Verabreichung des SM-Modulators unter Verwendung des Verfahrens von Anspruch 5, wobei die SM-Modulatoren wie in Anspruch 1 definiert sind.

7. Verfahren nach einem der Ansprüche 1 bis 6, wobei der SM-Modulator aus der Gruppe ausgewählt wird, bestehend aus Verbindungen #2-6, die die folgenden Strukturen aufweisen, und pharmazeutisch verträglichen Salzen davon:

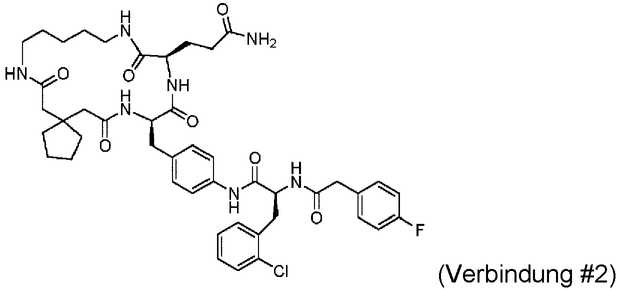

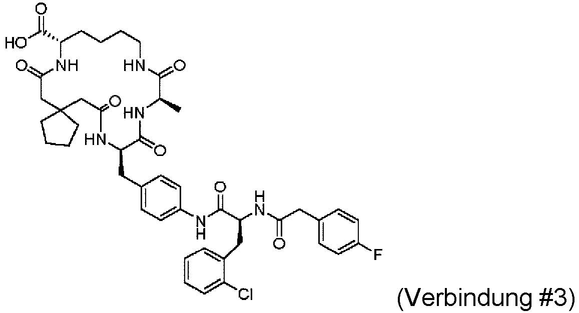

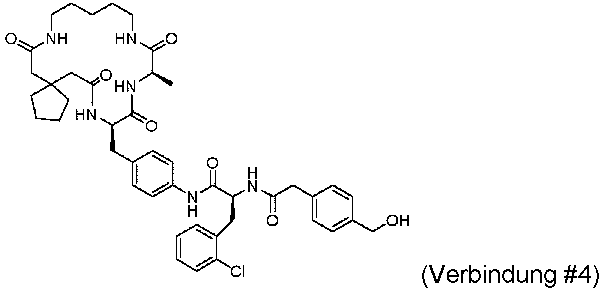

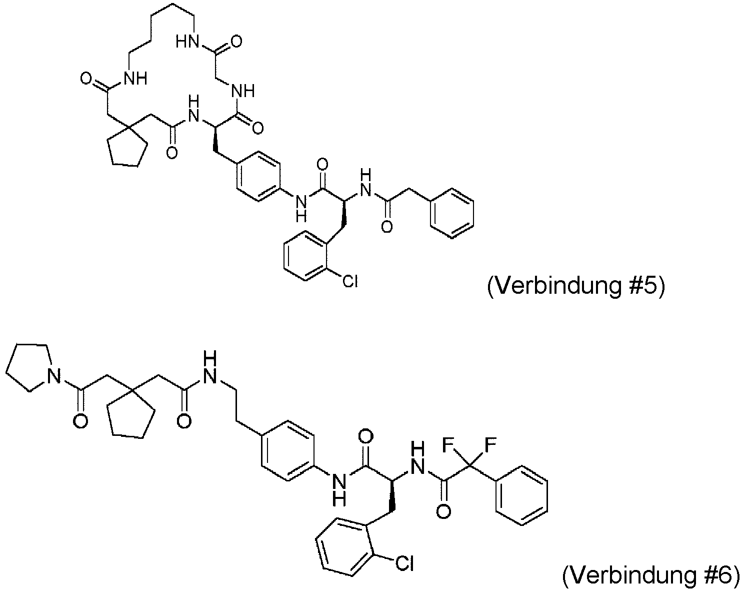

8. Verfahren nach einem der Ansprüche 1 bis 7, wobei das erste und/oder das zweite Nachweismittel mit einer nachweisbaren Markierung markiert ist, mehr bevorzugt, wobei die Markierung ein Enzym oder Biotin ist.

9. Verfahren nach einem der Ansprüche 5 bis 8, wobei der zweite Fängerantikörper und das zweite Nachweismittel aus den Paaren in der folgenden Tabelle ausgewählt werden:
| **Zweiter Fängerantikörper** | | **Zweites Nachweismittel** | |
|---|---|---|---|
| **Unternehmen** | **KAT#** | **Unternehmen** | **KAT#** |
| R&D Systems, Inc. | Fängerantikörper des DuoSet ELISA-Kits DY317 | R&D Systems, Inc. | Nachweisantikörper des Duo Set ELISA Kits DY317 |
| R&D Systems, Inc. | Fängerantikörper des DuoSet ELISA-Kits DY317 | R&D Systems, Inc. | Nachweisantikörper eines humanen IL-17 HS ELISA KitHS170 |
| R&D Systems, Inc. | Fängerantikörper des DuoSet ELISA-Kits DY317 | CovalAb R&D (Antikörper Online) | Antikörper ABIN1724556 |
| R&D Systems, Inc. | Fängerantikörper des DuoSet ELISA-Kits DY317 | Creative Diagnostics | Anti-IL 17A monoklonaler Antikörper CABT-ZC1184 |
| R&D Systems, Inc. | Fängerantikörper des DuoSet ELISA-Kits DY317 | Mabtech AB | Anti-humaner IL-17 Antikörper 3520-3-250 |
| R&D Systems, Inc. | Fängerantikörper des DuoSet ELISA-Kits DY317 | MyBioSource (MBS) | Anti-IL-17A-Antikörper MBS5 92444 |
| R&D Systems, Inc. | Fängerantikörper des DuoSet ELISA-Kits DY317 | MyBioSource (MBS) | Anti-IL-17A-Antikörper MBS592430 |
| R&D Systems, Inc. | Fängerantikörper des DuoSet ELISA-Kits DY317 | MyBioSource (MBS) | Anti-IL-17A-Antikörper MBS689609 |
| R&D Systems, Inc. | Fängerantikörper des DuoSet ELISA-Kits DY317 | MyBioSource (MBS) | Anti-IL-17A-Antikörper MBS592432 |
| R&D Systems, Inc. | Fängerantikörper des DuoSet ELISA-Kits DY317 | NOVUS Biologicals LLC | IL-17/IL-17A antibody DDX0336B-100 |
| R&D Systems, Inc. | Fängerantikörper des DuoSet ELISA-Kits DY317 | Antibodies Online | ABIN786722 |
| R&D Systems, Inc. | Fängerantikörper des DuoSet ELISA-Kits DY317 | OriGene Technologies Inc. | DDX0334B-50 |
| R&D Systems, Inc. | Fängerantikörper des DuoSet ELISA-Kits DY317 | OriGene Technologies Inc. | DDX0335B-50 |
| R&D Systems, Inc. | Fängerantikörper des DuoSet ELISA-Kits DY317 | Rockland Immunochemicals Inc. | IL-17A-Biotin-konjugierter Antikörper 209-306-B32 |
optional wobei der zweite Fängerantikörper und das zweite Nachweismittel Fängerantikörper beziehungsweise Nachweisantikörper des DuoSet ELISA-Kits DY317 (von R&D Systems, Inc.) sind.

10. Verfahren nach einem der Ansprüche 1 bis 9, wobei:
a. die Probe eine biologische Probe ist, die von einem Subjekt unter einer ex*vivo-* oder in-vivo-Behandlung mit dem SM-Modulator erhalten wird, optional wobei:
i. das Subjekt ein Säugetier, vorzugsweise ein Mensch ist; und/oder
ii. die biologische Probe aus der Gruppe ausgewählt wird, bestehend aus einer Probe, die aus Zellen, Geweben oder einer Serum-, Plasma- oder anderen biologischen Fluidprobe hergestellt wird; und/oder
b. das Verfahren eine Nachweisempfindlichkeit mit einer unteren Quantifizierungsgrenze (LLOQ) von etwa 0,01 oder etwa 0,1 oder etwa 0,25 oder etwa 0,5 oder etwa 0,75 oder etwa 1 pg/ml des IL-17 in der Probe aufweist.

11. Kit zum Bestimmen einer Menge an freiem IL-17 in einer Probe, umfassend IL-17 und einen SM-Modulator von IL-17, das Kit umfassend:
i. einen ersten Fängerantikörper, wobei bei dem Inkontaktbringen mit der Probe, der erste Fängerantikörper einen ersten Komplex ausbildet, umfassend den ersten Fängerantikörper und das IL-17, das an den SM-Modulator ungebunden ist, und einen zweiten Komplex, umfassend den ersten Fängerantikörper und das IL-17, das an den SM-Modulator gebunden ist; und
ii. ein erstes Nachweismittel, wobei bei dem Inkontaktbringen mit dem ersten Komplex und dem zweiten Komplex, das erste Nachweismittel einen dritten Komplex ausbildet, umfassend den ersten Fängerantikörper, das IL-17, das an den SM-Modulator ungebunden ist und das erste Nachweismittel, jedoch keinen vierten Komplex, umfassend den ersten Fängerantikörper, das IL-17, das an den SM-Modulator gebunden ist, und das erste Nachweismittel,
wobei der SM-Modulator aus der Gruppe ausgewählt ist, bestehend aus Verbindungen #1-6, die die folgenden Strukturen aufweisen, und pharmazeutisch verträglichen Salzen davon: 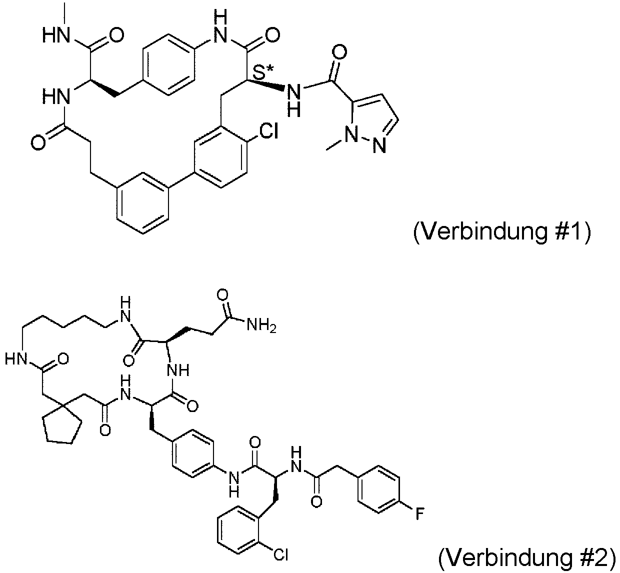 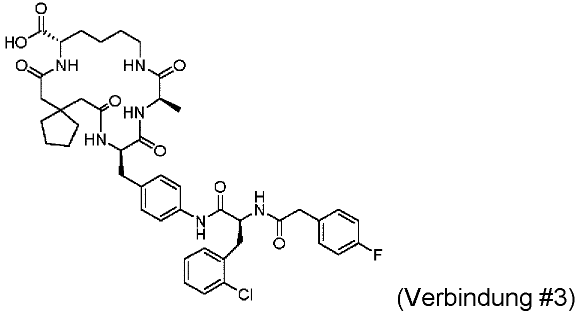 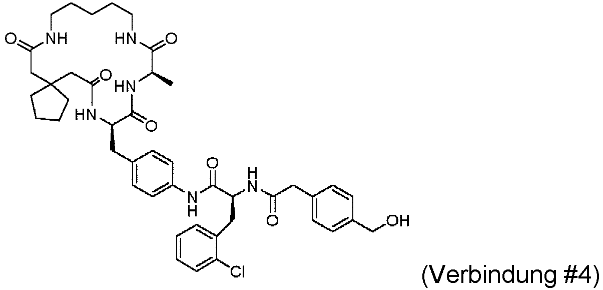 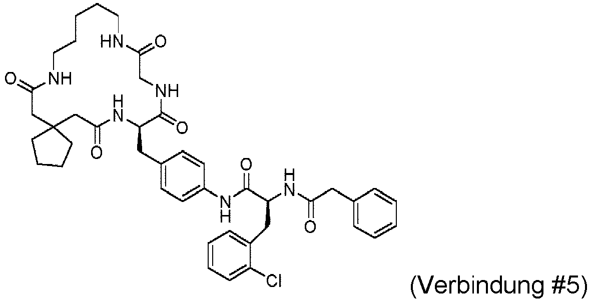 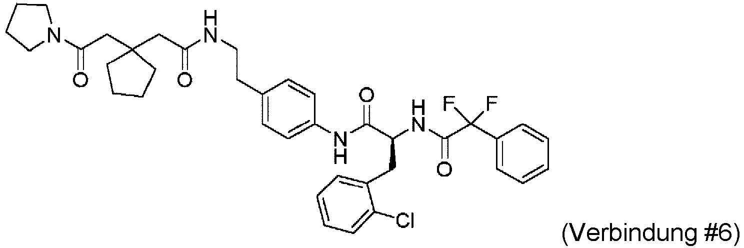
der erste Fängerantikörper der Fängerantikörper des DuoSet ELISA-Kits DY317 (R&D Systems, Inc.) ist und das erste Nachweismittel mAb4538 (Janssen Biotech, Inc.) ist und das erste Nachweismittel nicht in der Lage ist, einen Komplex auszubilden, umfassend den ersten Fängerantikörper, das IL-17, das an eine beliebige der Verbindungen #1 bis #6 gebunden ist und das erste Nachweismittel.

12. Kit nach Anspruch 11, ferner umfassend:
B. einen zweiten Fängerantikörper, wobei bei dem Inkontaktbringen mit der Probe, der zweite Fängerantikörper einen fünften Komplex ausbildet, umfassend den zweiten Fängerantikörper und das IL-17, das an den SM-Modulator ungebunden ist, und einen sechsten Komplex, umfassend den zweiten Fängerantikörper und das IL-17, das an den SM-Modulator gebunden ist; und
C. ein zweites Nachweismittel, wobei bei dem Inkontaktbringen mit dem fünften Komplex und dem sechsten Komplex, das zweite Nachweismittel einen siebten Komplex ausbildet, umfassend den zweiten Fängerantikörper, das IL-17, das an den SM-Modulator ungebunden ist und das zweite Nachweismittel, und einen achten Komplex, umfassend den zweiten Fängerantikörper, das IL-17, das an den SM-Modulator gebunden ist beziehungsweise das zweite Nachweismittel.

13. Kit nach Anspruch 12, wobei das erste Nachweismittel und/oder das zweite Nachweismittel mit einer nachweisbaren Markierung markiert ist, mehr bevorzugt, wobei die Markierung ein Enzym oder Biotin ist.

14. Kit nach einem der Ansprüche 12 bis 13, wobei der zweite Fängerantikörper und das zweite Nachweismittel aus den Paaren in der folgenden Tabelle ausgewählt werden:
| **Zweiter Fängerantikörper** | | **Zweites Nachweismittel** | |
|---|---|---|---|
| **Unternehmen** | **KAT#** | **Unternehmen** | **KAT#** |
| R&D Systems, Inc. | Fängerantikörper des DuoSet ELISA-Kits DY317 | R&D Systems, Inc. | Nachweisantikörper des Duo Set ELISA Kits DY317 |
| R&D Systems, Inc. | Fängerantikörper des DuoSet ELISA-Kits DY317 | R&D Systems, Inc. | Nachweisantikörper eines humanen IL-17 HS ELISA KitHS170 |
| R&D Systems, Inc. | Fängerantikörper des DuoSet ELISA-Kits DY317 | CovalAb R&D (Antikörper Online) | Antikörper ABIN1724556 |
| R&D Systems, Inc. | Fängerantikörper des DuoSet ELISA-Kits DY317 | Creative Diagnostics | Anti-IL 17A monoklonaler Antikörper CABT-ZC1184 |
| R&D Systems, Inc. | Fängerantikörper des DuoSet ELISA-Kits DY317 | Mabtech AB | Anti-humaner IL-17 Antikörper 3520-3-250 |
| R&D Systems, Inc. | Fängerantikörper des DuoSet ELISA-Kits DY317 | MyBioSource (MBS) | Anti-IL-17A-Antikörper MBS5 92444 |
| R&D Systems, Inc. | Fängerantikörper des DuoSet ELISA-Kits DY317 | MyBioSource (MBS) | Anti-IL-17A-Antikörper MBS592430 |
| R&D Systems, Inc. | Fängerantikörper des DuoSet ELISA-Kits DY317 | MyBioSource (MBS) | Anti-IL-17A-Antikörper MBS689609 |
| R&D Systems, Inc. | Fängerantikörper des DuoSet ELISA-Kits DY317 | MyBioSource (MBS) | Anti-IL-17A-Antikörper MBS592432 |
| R&D Systems, Inc. | Fängerantikörper des DuoSet ELISA-Kits DY317 | NOVUS Biologicals LLC | IL-17/IL-17A antibody DDX0336B-100 |
| R&D Systems, Inc. | Fängerantikörper des DuoSet ELISA-Kits DY317 | Antibodies Online | ABIN786722 |
| R&D Systems, Inc. | Fängerantikörper des DuoSet ELISA-Kits DY317 | OriGene Technologies Inc. | DDX0334B-50 |
| R&D Systems, Inc. | Fängerantikörper des DuoSet ELISA-Kits DY317 | OriGene Technologies Inc. | DDX0335B-50 |
| R&D Systems, Inc. | Fängerantikörper des DuoSet ELISA-Kits DY317 | Rockland Immunochemicals Inc. | IL-17A-Biotin-konjugierter Antikörper 209-306-B32 |
, optional wobei der zweite Fängerantikörper und das zweite Nachweismittel Fängerantikörper beziehungsweise Nachweisantikörper des DuoSet ELISA-Kits DY317 (von R&D Systems, Inc.) sind.

## Revendications

1. Procédé de détermination d'une quantité d'IL-17 libre dans un échantillon comprenant de l'IL-17 et un modulateur à petites molécules (SM) d'IL-17, le procédé comprenant :
i. la mise en contact de l'échantillon avec un premier anticorps de capture pour former un mélange comprenant un premier complexe comprenant le premier anticorps de capture et l'IL-17 non liée au modulateur SM, et un deuxième complexe comprenant le premier anticorps de capture et l'IL-17 liée au modulateur SM ;
ii. la mise en contact du mélange de l'étape i) avec un premier agent de détection pour former un troisième complexe comprenant le premier anticorps de capture, l'IL-17 non liée au modulateur SM et le premier agent de détection, mais pas un quatrième complexe comprenant le premier anticorps de capture, l'IL-17 liée au modulateur SM et le premier agent de détection ; et
iii. la détermination de la quantité d'IL-17 libre en mesurant la quantité du premier agent de détection dans le troisième complexe,
dans lequel le modulateur SM est choisi dans le groupe constitué des composés #1 à 6 ayant les structures suivantes et des sels pharmaceutiquement acceptables de ceux-ci : 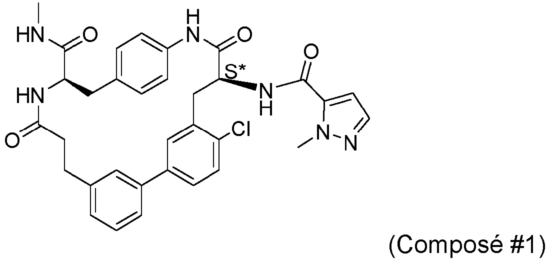 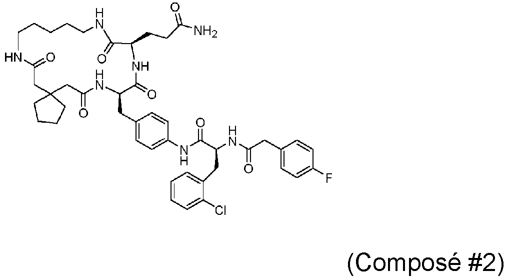 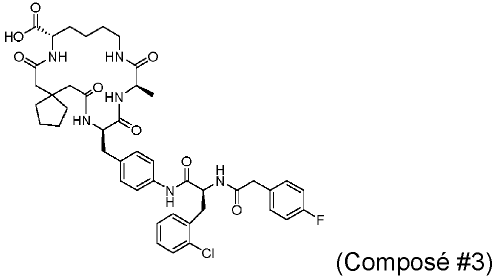 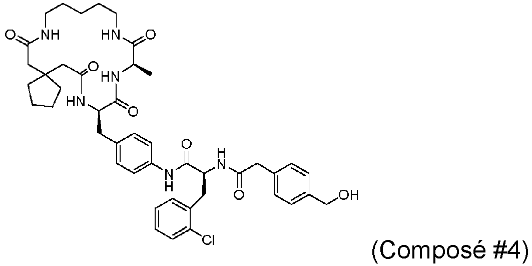 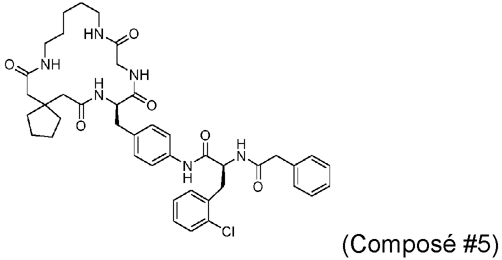 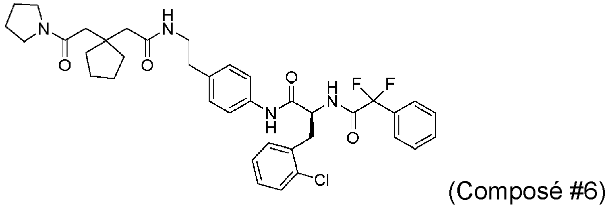
le premier anticorps de capture est l'anticorps de capture du kit DuoSet ELISA DY317 (R&D Systems, Inc.) et le premier agent de détection est le mAb4538 (Janssen Biotech, Inc.) et le premier agent de détection n'est pas capable de former un complexe comprenant le premier anticorps de capture, l'IL-17 liée à l'un des composés #1 à #6 et le premier agent de détection.

2. Procédé selon la revendication 1, dans lequel aucune étape de lavage n'est effectuée avant la mise en contact du mélange de l'étape i) avec le premier agent de détection.

3. Procédé de détermination de la prise cible d'un modulateur SM d'IL-17 chez un sujet, comprenant a) la détermination de la quantité d'IL-17 libre dans un échantillon provenant du sujet avant l'administration du modulateur SM à l'aide du procédé selon la revendication 1 ; b) la détermination de la quantité d'IL-17 libre dans un échantillon provenant du sujet après l'administration du modulateur SM à l'aide du procédé selon la revendication 1 ; et c) la comparaison des quantités d'IL-17 libre déterminées aux étapes a) et b), dans lequel les modulateurs SM sont selon la revendication 1.

4. Procédé de détermination de la prise cible d'un modulateur SM d'IL-17 chez un sujet, comprenant a) la détermination de la quantité d'IL-17 libre dans un échantillon provenant du sujet à deux points de temps ou plus après l'administration du modulateur SM à l'aide du procédé selon la revendication 1 ; et b) la comparaison des quantités d'IL-17 libre aux deux points de temps ou plus déterminées à l'étape a), dans lequel les modulateurs SM sont selon la revendication 1.

5. Procédé de détermination d'une quantité d'IL-17 liée à un modulateur dans un échantillon comprenant de l'IL-17 et un modulateur SM d'IL-17, le procédé comprenant :
A. la détermination de la quantité d'IL-17 libre à l'aide du procédé selon la revendication 1 ;
B. la mise en contact d'une seconde partie de l'échantillon avec un second anticorps de capture pour former un second mélange comprenant un cinquième complexe comprenant le second anticorps de capture et l'IL-17 non liée au modulateur SM, et un sixième complexe comprenant le second anticorps de capture et l'IL-17 liée au modulateur SM ;
C. la mise en contact du second mélange avec un second agent de détection pour former un septième complexe comprenant le second anticorps de capture, l'IL-17 non liée au modulateur SM et le second agent de détection, et un huitième complexe comprenant le second anticorps de capture, l'IL-17 liée au modulateur SM et le second agent de détection ;
D. la détermination d'une quantité totale d'IL-17 en mesurant la quantité totale du second agent de détection dans le septième complexe et le huitième complexe ; et
E. la détermination de la quantité d'IL-17 liée au modulateur dans l'échantillon en soustrayant la quantité d'IL-17 libre déterminée à l'étape (A) de la quantité totale d'IL-17 à l'étape (D), dans lequel les modulateurs SM sont selon la revendication 1.

6. Procédé de détermination de la prise cible d'un modulateur SM d'IL-17 chez un sujet, comprenant la détermination de la quantité d'IL-17 liée au modulateur dans un échantillon provenant du sujet après l'administration du modulateur SM à l'aide du procédé selon la revendication 5, dans lequel les modulateurs SM sont selon la revendication 1.

7. Procédé selon l'une quelconque des revendications 1 à 6, dans lequel le modulateur SM est choisi dans le groupe constitué des composés #2 à 6 ayant les structures suivantes et des sels pharmaceutiquement acceptables de ceux-ci :

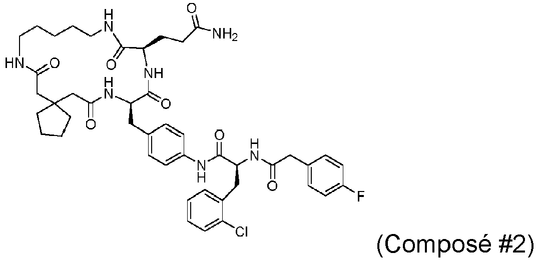

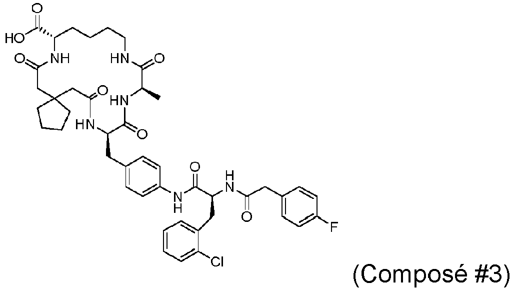

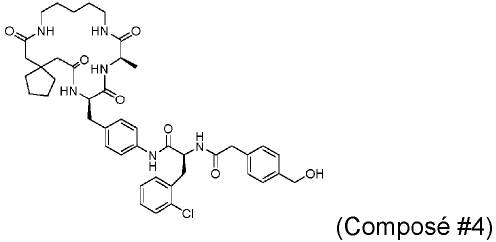

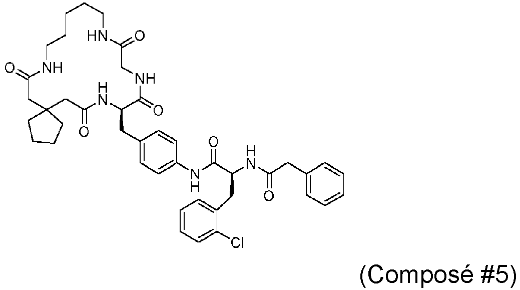

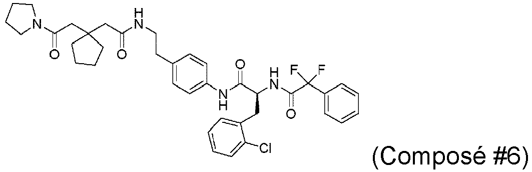

8. Procédé selon l'une quelconque des revendications 1 à 7, dans lequel le premier et/ou le second agent de détection est étiqueté avec une étiquette détectable, plus préférablement, l'étiquette est une enzyme ou de la biotine.

9. Procédé selon l'une quelconque des revendications 5 à 8, dans lequel le second anticorps de capture et le second agent de détection sont choisis parmi les paires du tableau suivant :
| **Second anticorps de capture** | | **Second agent de détection** | |
|---|---|---|---|
| **Entreprise** | **CAT#** | **Entreprise** | **CAT#** |
| R&D Systems, Inc. | Anticorps de capture du kit ELISA DuoSet DY317 | R&D Systems, Inc. | Anticorps de détection du kit ELISA Duo Set DY317 |
| R&D Systems, Inc. | Anticorps de capture du kit ELISA DuoSet DY317 | R&D Systems, Inc. | Anticorps de détection du kit ELISA HS IL-17 humain HS170 |
| R&D Systems, Inc. | Anticorps de capture du kit ELISA DuoSet DY317 | CovalAb R&D (Antibodies Online) | anticorps ABIN1724556 |
| R&D Systems, Inc. | Anticorps de capture du kit ELISA DuoSet DY317 | Creative Diagnostics | Anticorps monoclonal anti-IL 17A CABT-ZC1184 |
| R&D Systems, Inc. | Anticorps de capture du kit ELISA DuoSet DY317 | Mabtech AB | Anticorps anti IL-17 humain 3520-3-250 |
| R&D Systems, Inc. | Anticorps de capture du kit ELISA DuoSet DY317 | MyBioSource (MBS) | Anticorps anti-IL-17A MBS5 92444 |
| R&D Systems, Inc. | Anticorps de capture du kit ELISA DuoSet DY317 | MyBioSource (MBS) | Anticorps anti-IL-17A MBS592430 |
| R&D Systems, Inc. | Anticorps de capture du kit ELISA DuoSet DY317 | MyBioSource (MBS) | Anticorps anti-IL-17A MBS689609 |
| R&D Systems, Inc. | Anticorps de capture du kit ELISA DuoSet DY317 | MyBioSource (MBS) | Anticorps anti-IL-17A MBS592432 |
| R&D Systems, Inc. | Anticorps de capture du kit ELISA DuoSet DY317 | NOVUS Biologicals LLC | Anticorps IL-17/IL-17A DDX0336B-100 |
| R&D Systems, Inc. | Anticorps de capture du kit ELISA DuoSet DY317 | Antibodies Online | ABIN786722 |
| R&D Systems, Inc. | Anticorps de capture du kit ELISA DuoSet DY317 | OriGene Technologies Inc. | DDX0334B-50 |
| R&D Systems, Inc. | Anticorps de capture du kit ELISA DuoSet DY317 | OriGene Technologies Inc. | DDX0335B-50 |
| R&D Systems, Inc. | Anticorps de capture du kit ELISA DuoSet DY317 | Rockland Immunochemicals Inc. | Anticorps IL-17A conjugué à la biotine 209-306-B32 |
, éventuellement, dans lequel le second anticorps de capture et le second agent de détection sont respectivement l'anticorps de capture et l'anticorps de détection du kit DuoSet ELISA DY317 (de R&D Systems, Inc.).

10. Procédé selon l'une quelconque des revendications 1 à 9, dans lequel :
a. l'échantillon est un échantillon biologique obtenu à partir d'un sujet soumis à un traitement ex *vivo* ou *in vivo* avec le modulateur SM, éventuellement dans lequel :
i. le sujet est un mammifère, de préférence humain ; et/ou
ii. l'échantillon biologique est choisi dans le groupe constitué d'un échantillon préparé à partir de cellules, de tissus, ou d'un échantillon de sérum, de plasma ou d'un autre fluide biologique ; et/ou
b. le procédé a une sensibilité de détection avec une limite inférieure de quantification (LLOQ) à environ 0,01 ou environ 0,1 ou environ 0,25, ou environ 0,5, ou environ 0,75, ou environ 1 pg/ml d'IL-17 dans l'échantillon.

11. Kit pour déterminer une quantité d'IL-17 libre dans un échantillon comprenant de l'IL-17 et un modulateur SM d'IL-17, le kit comprenant :
i. un premier anticorps de capture, dans lequel, au contact de l'échantillon, le premier anticorps de capture forme un premier complexe comprenant le premier anticorps de capture et l'IL-17 non liée au modulateur SM, et un second complexe comprenant le premier anticorps de capture et l'IL-17 liée au modulateur SM ; et
ii. un premier agent de détection, dans lequel, au contact du premier complexe et du deuxième complexe, le premier agent de détection forme un troisième complexe comprenant le premier anticorps de capture, l'IL-17 non liée au modulateur SM et le premier agent de détection, mais pas un quatrième complexe comprenant le premier anticorps de capture, l'IL-17 liée au modulateur SM et le premier agent de détection,
dans lequel le modulateur SM est choisi dans le groupe constitué des composés #1 à 6 ayant les structures suivantes et des sels pharmaceutiquement acceptables de ceux-ci : 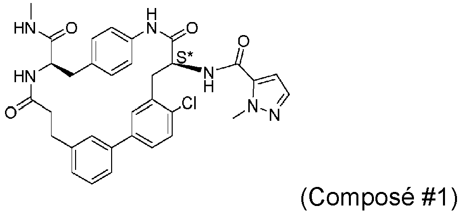 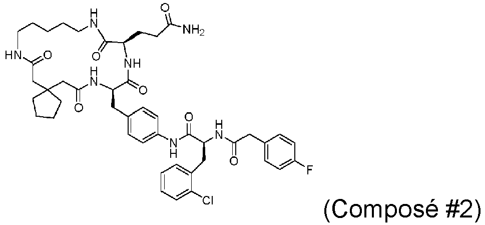 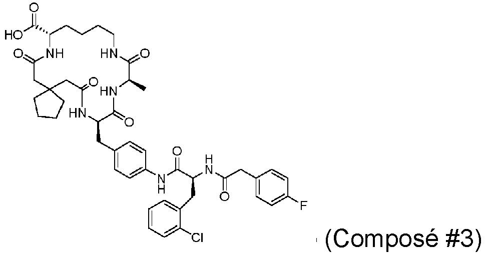 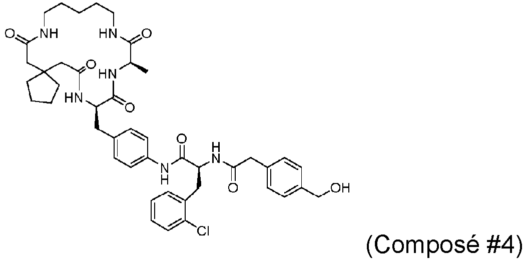 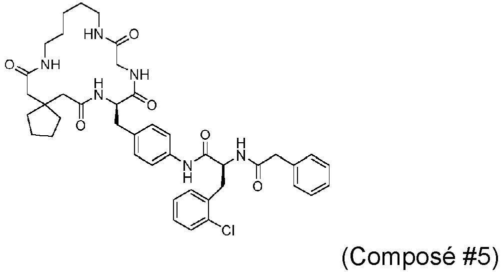 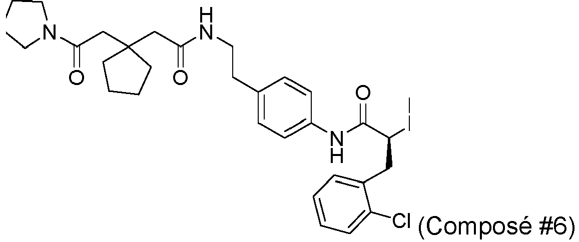
le premier anticorps de capture est l'anticorps de capture du kit DuoSet ELISA DY317 (R&D Systems, Inc.) et le premier agent de détection est le mAb4538 (Janssen Biotech, Inc.) et le premier agent de détection n'est pas capable de former un complexe comprenant le premier anticorps de capture, l'IL-17 liée à l'un quelconque des composés #1 à #6 et le premier agent de détection.

12. Kit selon la revendication 11, comprenant en outre :
B. un second anticorps de capture, dans lequel, au contact de l'échantillon, le second anticorps de capture forme un cinquième complexe comprenant le second anticorps de capture et l'IL-17 non liée au modulateur SM, et un sixième complexe comprenant le second anticorps de capture et l'IL-17 liée au modulateur SM ; et
C. un second agent de détection, dans lequel, au contact du cinquième complexe et du sixième complexe, le second agent de détection forme un septième complexe comprenant le second anticorps de capture, l'IL-17 non liée au modulateur SM et le second agent de détection, et un huitième complexe comprenant le second anticorps de capture, l'IL-17 liée au modulateur SM et le second agent de détection, respectivement.

13. Kit selon la revendication 12, dans lequel le premier agent de détection et/ou le second agent de détection est étiqueté avec une étiquette détectable, plus préférablement, l'étiquette est une enzyme ou de la biotine.

14. Kit selon l'une quelconque des revendications 12 à 13, dans lequel le second anticorps de capture et le second agent de détection sont choisis parmi les paires du tableau suivant :
| **Second anticorps de capture** | | **Second agent de détection** | |
|---|---|---|---|
| **Entreprise** | **CAT#** | **Entreprise** | **CAT#** |
| R&D Systems, Inc. | Anticorps de capture du kit ELISA DuoSet DY317 | R&D Systems, Inc. | Anticorps de détection du kit ELISA Duo Set DY317 |
| R&D Systems, Inc. | Anticorps de capture du kit ELISA DuoSet DY317 | R&D Systems, Inc. | Anticorps de détection du kit ELISA HS IL-17 humain HS170 |
| R&D Systems, Inc. | Anticorps de capture du kit ELISA DuoSet DY317 | CovalAb R&D (Antibodies Online) | anticorps ABIN1724556 |
| R&D Systems, Inc. | Anticorps de capture du kit ELISA DuoSet DY317 | Creative Diagnostics | Anticorps monoclonal anti-IL 17A CABT-ZC1184 |
| R&D Systems, Inc. | Anticorps de capture du kit ELISA DuoSet DY317 | Mabtech AB | Anticorps anti IL-17 humain 3520-3-250 |
| R&D Systems, Inc. | Anticorps de capture du kit ELISA DuoSet DY317 | MyBioSource (MBS) | Anticorps anti-IL-17A MBS5 92444 |
| R&D Systems, Inc. | Anticorps de capture du kit ELISA DuoSet DY317 | MyBioSource (MBS) | Anticorps anti-IL-17A MBS592430 |
| R&D Systems, Inc. | Anticorps de capture du kit ELISA DuoSet DY317 | MyBioSource (MBS) | Anticorps anti-IL-17A MBS689609 |
| R&D Systems, Inc. | Anticorps de capture du kit ELISA DuoSet DY317 | MyBioSource (MBS) | Anticorps anti-IL-17A MBS592432 |
| R&D Systems, Inc. | Anticorps de capture du kit ELISA DuoSet DY317 | NOVUS Biologicals LLC | Anticorps IL-17/IL-17A DDX0336B-100 |
| R&D Systems, Inc. | Anticorps de capture du kit ELISA DuoSet DY317 | Antibodies Online | ABIN786722 |
| R&D Systems, Inc. | Anticorps de capture du kit ELISA DuoSet DY317 | OriGene Technologies Inc. | DDX0334B-50 |
| R&D Systems, Inc. | Anticorps de capture du kit ELISA DuoSet DY317 | OriGene Technologies Inc. | DDX0335B-50 |
| | | | |
|---|---|---|---|
| R&D Systems, Inc. | Anticorps de capture du kit ELISA DuoSet DY317 | Rockland Immunochemical s Inc. | Anticorps IL-17A conjugué à la biotine 209-306-B32 |
, éventuellement, dans lequel le second anticorps de capture et le second agent de détection sont respectivement l'anticorps de capture et l'anticorps de détection du kit DuoSet ELISA DY317 (de R&D Systems, Inc.).
